(19)

Europäisches
Patentamt
European
Patent Office
Office européen
des brevets

(11) **EP 4 379 726 A1**

(12) **EUROPEAN PATENT APPLICATION**

(43) Date of publication:
**05.06.2024 Bulletin 2024/23**

(21) Application number: **22461636.7**

(22) Date of filing: **02.12.2022**

(51) International Patent Classification (IPC):
**G16B 35/20** *(2019.01)*     **G16B 40/20** *(2019.01)*

(52) Cooperative Patent Classification (CPC):
**G16B 35/20; G16B 40/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Ardigen S.A.**
**30-394 Krakow (PL)**

(72) Inventors:
- **Pienkowski Murcia, Victor**
  **03-028 Warszawa (PL)**
- **Jasinski, Maciej**
  **02-157 Warszawa (PL)**
- **Skoczylas, Piotr**
  **30-363 Krakow (PL)**
- **Wojciechowski, Daniel**
  **30-410 Krakow (PL)**
- **Mazzocco, Giovanni**
  **02-791 Warszawa (PL)**
- **Niemiec, Iga**
  **30-383 Krakow (PL)**
- **Sanecka-Duin, Anna**
  **30-377 Krakow (PL)**
- **Blum, Agnieszka**
  **31-503 Krakow (PL)**
- **Kaczmarczyk, Jan**
  **30-394 Krakow (PL)**
- **Krol, Paulina**
  **30-668 Krakow (PL)**
- **Myronov, Oleksandr**
  **30-383 Krakow (PL)**
- **Stachura, Slawomir**
  **30-316 Krakow (PL)**
- **Marczynska-Grzelak, Joanna**
  **30-392 Krakow (PL)**
- **Stepniak, Piotr**
  **62-052 Komorniki (PL)**

(74) Representative: **Patpol Kancelaria Patentowa Sp. z o.o.**
**Nowoursynowska 162J**
**02-776 Warszawa (PL)**

Remarks:
The complete document including Reference Table(s) and the Sequence Listing(s) can be downloaded from the EPO website

(54) **THE METHOD FOR PREDICTING THE OCCURRENCE OF THE OFF-TARGET TOXICITY CAUSED BY SIMILARITY BETWEEN A TARGET EPITOPE AND PUTATIVE OFF-TARGET EPITOPES**

(57)     The subject of the invention is a computer implemented method for predicting the occurrence of the off-target toxicity caused by similarity between a target epitope and off-target epitopes, the method comprising: a) providing said target epitope of a predefined length and its corresponding HLA type, b) identifying a set of all putative off-target epitopes present in a reference proteome having the same length as the predefined length of said target epitope, c) extracting a subset of off-target epitopes from the identified set of all putative off-target epitopes having surface HLA presentation probability for said HLA type above a presentation probability threshold value with the use of a presentation model, d) ranking said extracted subset of off-target epitopes for its potential for toxicity by calculating a numerical score value for each off-target epitope indicative of a degree of similarity between said target epitope and each said off-target epitope, based on at least one physico-chemical property of the amino acids at predefined fixed positions interacting with antigen-recognition receptors.

EP 4 379 726 A1

**(Cont. next page)**

**INPUT**

- target epitope
- HLA type

Search of similiar sequences from the human reference proteome

Addition of frequent SNP-derived epitopes

Removal of off-target epitopes based on pHLA presentation and binding

Similarity scoring of off-target epitopes based on TCR faced amino acids

Similarity scoring of off-target epitopes based on TCR faced amino acids

Addition of mRNA and peptide tissue specific expression data for each off-target epitope

**OUTPUT**

Table with prioritized and scored cross-reactive antigens and their expression patterns

**Fig. 1**

**Description**

FIELD OF THE INVENTION

The present invention relates to a method for predicting the occurrence of the off-target toxicity caused by similarity between a target epitope and putative off-target epitopes.

BACKGROUND OF THE INVENTION

Recent advances in the field of cancer immunotherapy have significantly changed the landscape of available treatments, thus improving the prognosis for many patients with various hematological and solid malignancies. Leveraging the capacity of the immune system to differentiate tumor antigens from natural ones has already been shown to lead to sustained clinical responses. Cell-based approaches for boosting natural defenses include: inter alia, Tumor-Infiltrating Lymphocytes (TILs), Engineered T Cell Receptor (TCR) or Chimeric Antigen Receptor (CAR) modified cells. Adoptive cell therapies have a high potential for revolutionizing cancer treatment; however, despite their immense potential, they are not free of side effects. The immune system, while being revved up to fight the disease, might also act against healthy cells and tissues, even more so when T cells are genetically modified. The development of such off-target immunotoxicity can have serious health consequences for patients undergoing therapy and is a major clinical concern, as fatalities in clinical trials have been reported.

In humans T-cells undergo positive and negative selection that results in survival of T-cells that moderately bind to HLA and do not bind to self antigens (see Klein L, Kyewski B, Allen PM, Hogquist KA. Positive and negative selection of the T cell repertoire: what thymocytes see (and don't see. Nat Rev Immunol. 2014 Jun;14(6):377-91. doi: 10.1038/nri3667. Epub 2014 May 16.) This results in an adaptive immune system that acquires the ability to distinguish tumor cells from healthy ones. Immunosurveillance by T lymphocytes is possible through the recognition of foreign epitopes that are presented on the cell's surface. Interestingly, most T cells can be activated by more than one epitope. The ability to identify all epitopes that can activate a given T cell is one of the main challenges in the development of cellular immunotherapies. Experimental approaches to this problem are far from perfect as they are limited to a restricted subspace of potential off-targets' sequences. Furthermore, probing the entire sequence space experimentally is unfeasible due to time and cost limitations. On the other hand, the ability to quickly find all potential off-target epitopes is highly desirable as off-target toxicity has already proven to be extremely dangerous to patient's health because it resulted in the death of several people in clinical trials.

Most of the prior art in vitro approaches that determine off-target toxicity often require screening of protein sequence databases for peptides with similarity to the target epitope. However, these solutions often return a large number of peptides, all of which would need to be tested experimentally with no assurance of successful identification of the off-target epitope. Lowering the stringency of the search parameters would further increase the number of potential epitopes that would have to be tested and again may still not reveal off-target peptides.

For example, patent document US11 017882B2 discloses a method for predicting whether a binding peptide, that binds a target peptide-MHC complex, has the potential to cross react in vivo with another peptide in the subject so as to cause unwanted side effects. The method comprises the following steps: identification of at least one motif in the target peptide to which the binding peptide binds, search for peptides that are present in the subject that contain at least one previously identified binding motif in a peptide that is not the target, and test the binding of any such peptide to the binding peptide in vitro. The presence of one or more such peptides indicates that the binding peptide has the potential to cross react in vivo.

Another in vitro method of screening and identifying T cell epitopes is disclosed in the patent application US2019227063A1. Said method can be used to predict and study the toxicity and off-target effects of T-cells, TCRs, or TCR-like molecules. This approach requires contacting an engineered target cell with a T cell, a TCR, or a TCR-like molecule, and performing an assay to determine whether the T cell, TCR, or a TCR-like molecule binds to the engineered target cell, and to measure the strength of any such binding. Even though said approach extensively studies TCR:pHLA interactions, and has numerous advantages over the prior art methods (inter alia can utilize mammalian cells, do not require covalent linkage of MHC molecules to the peptides displayed on the MHC molecules, and can allow precisely defined HLA-presentable antigens to be expressed), it requires a vast amount of time and financial resources to be conducted.

In parallel, many other prior art methods use animal models to measure immune responses generated by the binding peptide (TCR) and target epitope interaction.

For example, the international application WO2019006427A1 discloses a mouse model for assessing toxicities associated with immunotherapies. The known method comprises of administering a lymphodepleting agent to an immunocompetent mouse, wherein the lymphodepleting agent or therapy does not comprise total body radiation and/or does not comprise complete or substantially complete immune ablation; and subsequently administering to the mouse an immunotherapy, wherein the immunotherapy binds to and/or recognizes an antigen that is expressed on or in a cell or tissue of the immunocompetent mouse. However, since the differences between human protein sequences and those of the animal exist, the absence of unwanted side effects in the animal may not translate to humans.

Therefore, it is desirable to provide a method for *in silico* predicting whether off-target epitopes exist in the human or organisms living in the human, that show a TCR-pHLA interaction similar to a specific target epitope. This would allow binding peptides directed against a target epitope to be chosen and designed in a way that they do not bind to off-target epitopes and consequently have a far greater chance of not causing unwanted side effects.

Such *in silico* prediction methods are known already in the prior art. For example, one method is disclosed in the publication "In-silico discovery of cancer-specific peptide-HLA complexes for targeted therapy 'by Dhanik et al. BMC Bioinformatics (2016) 17:286. Another cross reactivity prediction method is disclosed in Moise, L., Gutierrez, A. H., Kibria, F., Martin, R., Tassone, R., Liu, R., Terry, F., Martin, W., & De Groot, A. S. (2015). iVax: "An integrated toolkit for the selection and optimization of antigense and the design of epitope-driven vaccines". Human Vaccines & Immunotherapeutics, 11 (9), 2312-2321 and Moise L, Gutierrez AH, Bailey-Kellogg C, Terry F, Leng Q, Abdel Hady KM, VerBerkmoes NC, Sztein MB, Losikoff PT, Martin WD, Rothman AL, De Groot AS. The two-faced T cell epitope: examining the host-microbe interface with JanusMatrix. Hum Vaccin Immunother. 2013 Jul;9(7):1577-86.

However, known *in silico* off-target toxicity prediction methods still have room for improvement as both the accuracy and efficiency of such methods, can be further enhanced.

SUMMARY OF THE INVENTION

It is therefore desirable to provide a method for predicting the occurrence of the off-target toxicity caused by similarity between a target epitope and off-target epitopes, which addresses drawbacks of the prior art and provides better accuracy and enhanced efficiency.

The present invention provides a computer implemented method for predicting the occurrence of the off-target toxicity caused by similarity between a target epitope and off-target epitopes, which detects and ranks putative off-target toxicity accurately, while taking into account patient specific genomic characteristics.

In one aspect a computer implemented method a computer implemented method for predicting the occurrence of the off-target toxicity caused by similarity between a target epitope and off-target epitopes is provided, the method comprising the following steps: providing said target epitope of a predefined length and its corresponding HLA type, identifying a set of all putative off-target epitopes present in a reference proteome having the same length as the predefined length of said target epitope, extracting a subset of off-target epitopes from the identified set of all putative off-target epitopes having surface HLA presentation probability for said HLA type above a presentation probability threshold value with the use of a presentation model, ranking said extracted subset of off-target epitopes for its potential for toxicity by calculating a numerical score value for each off-target epitope indicative of a degree of similarity between said target epitope and each said off-target epitope, based on at least one physico-chemical property of the amino acids at predefined fixed positions interacting with antigen-recognition receptors.

Advantageously, said at least one physico-chemical property relates to at least one among: amino acid secondary structure prediction beta-strand, aperiodic structure and alpha helix, sizes of radii and volume of atomic groups, relation of amino acid composition depending on protein localization and inter-residue protein contact energies, advantageously at least one among: isoelectric point, polarity, molecular weight, average accessible surface area, mutability, hydration potential, refractivity, optical activity, flexibility.

Advantageously, defining fixed positions in amino acid sequence of the target epitope and the off-target epitope responsible for interacting with antigen-recognition receptors comprises consulting a residues database with amino acid positions important for interaction with antigen-recognition receptors depending on the HLA type relating to said target epitope and off-target epitope, said database being generated based on at least results of A-scan and/or X-scan.

Advantageously, said database with amino acid positions important for interaction with antigen-recognition receptors depending on the HLA type relating to said target epitope and off-target epitope is additionally generated with the data relating to non-HLA faced amino acids.

Moreover, advantageously, the step d) of ranking off-target epitopes by obtaining a numerical score value for each said off-target epitope comprises: limiting amino acids sequences for said target epitope and for said each off-target epitope by deleting amino acids in their sequences at predefined fixed positions that are not interacting with antigen-recognition receptors, for said target epitope and for said each off-target epitope determining a vector of the size n=pn * rn, where pn is the number of predefined residues and rn is the number of physico-chemical properties used for scoring, then calculating the distance x between said target epitope and said off-target epitope according to the following norm:

$$x = \sqrt{\sum_{i=1}^{n}(e_i - p_i)^2}$$

where $e_i$ and pi represent the *i-th* amino acids physico-chemical property from compared target epitope e and off-target epitope *p,* and *n* is equal to the length of the vector $n = p_n * r_n$, receiving the numerical score value x for each said off-target epitope being said calculated distance, wherein said numerical score x ranges between 0 and 40, advantageously between 0 and 14, wherein if the value of the numerical score value for the off-target under assessment is close to 0 it means that said off-target epitope and the target epitope are almost identical with high probability for toxicity to occur and wherein if the value of the safety score for the off-target under assessment is above a threshold value it means that the off-target under assessment has a low potential for toxicity.

Advantageously, the predefined length of the target epitope is between 8 to 11 amino acids.

Advantageously, the step b) of identifying a set of all putative off-target epitopes present in a reference proteome having the same length as the predefined length of the target epitope comprises: generating an artificial set of amino acids sequences having at least 5 amino acids in common with said target epitope according to a formula:

$$\sum_{k=0,1...n} \frac{N!}{(N!-k!)k!} * 20^{k}.$$

wherein N is the predefined length of the target epitope and k is the number of admissible mismatches in amino acids sequence, then checking occurrence of said artificially generated set of epitopes in the reference proteome, and receiving a set of all naturally existing putative off-target epitopes by selecting only those artificially generated off-target epitopes which are present in said reference proteome.

Advantageously before the step c) the method further comprises enlarging said set of all putative off-target epitopes through addition of epitopes derived from frequent genetic variants.

Advantageously, enlarging said set of all putative off-target epitopes through addition of epitopes derived from frequent genetic variants comprises for each off-target epitope within said of all putative off-target epitopes: determining an ENSG identificator based on its uniprot identifier, finding genome region, which codes said epitope using said ENSG identificator, along with the start and end position of the off-target epitope in the found genome region, consulting the gnomAD database for mutations in said genome region, transcripting each frequent mutation in said genome region into an additional naturally existing off-target epitope, receiving an enlarged set of all naturally existing putative off-target epitopes by adding said additional naturally existing epitopes resulting from said mutations.

More advantageously, the step c) further comprises a preceding or subsequent substep of selecting only a subset of off-target epitopes having binding affinity level below a binding affinity threshold value, advantageously the binding affinity threshold value is equal to the IC50 value equal to 2000 nM.

Advantageously, in the step c) the presentation probability threshold value is equal to 50%.

Advantageously, in the step c) said presentation model is a trained presentation model and is based on at least one artificial neural network, and wherein the model is trained using datasets consisting of mass-spectroscopy experiments results conducted on monoallelic human cell line.

Advantageously, the method further comprises the step of identifying mRNA and peptide tissue specific expression for each gene derived from presented off-target epitope within said extracted subset along with indication of tissue sensitive to off-target toxicity caused by said each off-target epitope.

In another aspect, it is provided a computer-readable storage medium having instructions encoded thereon which when executed by a processor, cause the processor to perform the method according to the invention.

Yet, in another aspect, it is provided a computer system having at least one processor and one memory configured to perform the method according to the invention.

By generating artificially all possible amino acid sequence combinations with a predefined maximum number of mismatches in relation to the target epitope, all logically possible cases are taken into account for prediction, thus making the method more credible, robust and accurate.

By enlarging the set of putative off-target epitopes through addition of frequently present SNPs, all possible naturally existing putative off-target epitopes that are not present in the reference genome undergo the process of prediction their cross-reactivity, thus again making the method more credible, robust and accurate and patient specific.

In particular, the sensitivity and accuracy of the toxicity prediction is enhanced by analyzing, as a separate step, possible interaction of putative off-target epitopes with the TCR. Selection of TCR residues based on data gathered from widespread studies allows for choosing for further analysis only amino acids which might have a substantial impact on said interaction. As a consequence, the method is again more credible and robust but also more efficient.

Moreover, by introducing an automated and mathematically based ranking method of off-target toxicity probability which again uses pre-selected and aggregated sets of data from different studies relating to physico-chemical properties, the method better reflects real phenomena, which enhances its credibility.

The method according to the invention has better efficiency also because of the use of the presentation model based on artificial neural networks and trained on datasets from mass-spectroscopy.

Finally, the results of the method according to the invention are outputted in a complex manner, namely by associating obtained off-target epitopes not only with their toxicity safety score but also with expression patterns of the gene derived from off-target epitope in question for mRNA and peptides. This results in comprehensive understanding of the putative off-target epitopes and enables the user to swiftly move from in silico analysis to in vitro validation.

BRIEF DESCRIPTION OF THE DRAWINGS

The following detailed description, given by way of example, but not intended to limit the invention solely to the specific embodiments described, may best be understood in conjunction with the accompanying drawings, wherein:

Fig. 1 shows a workflow diagram depicting consecutive steps of the method according to the invention;
Fig. 2 shows a detailed pipeline relating to the step of enlarging a set of naturally existing putative off-target epitopes resulting from frequent genetic variants;

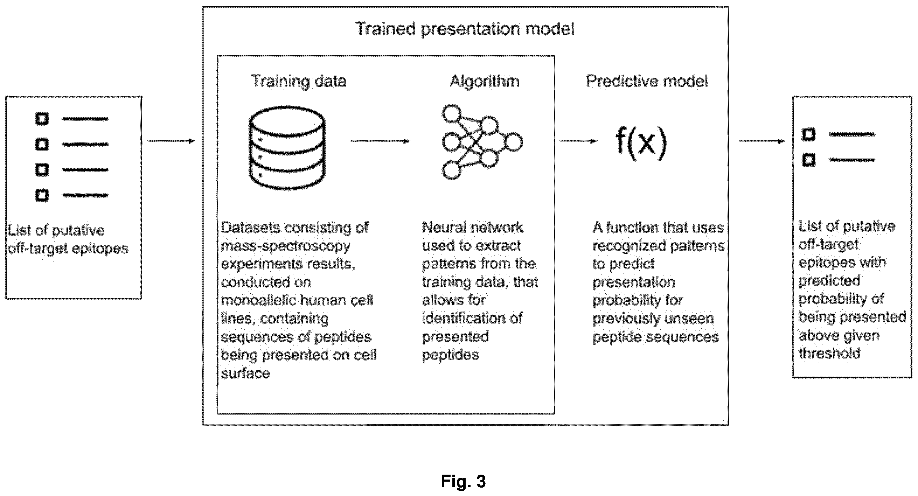

Fig.3 shows a detailed pipeline relating to the step of selecting from the enlarged set only putative off-target epitopes that have high probability of being presented on the cell surface with a trained model;

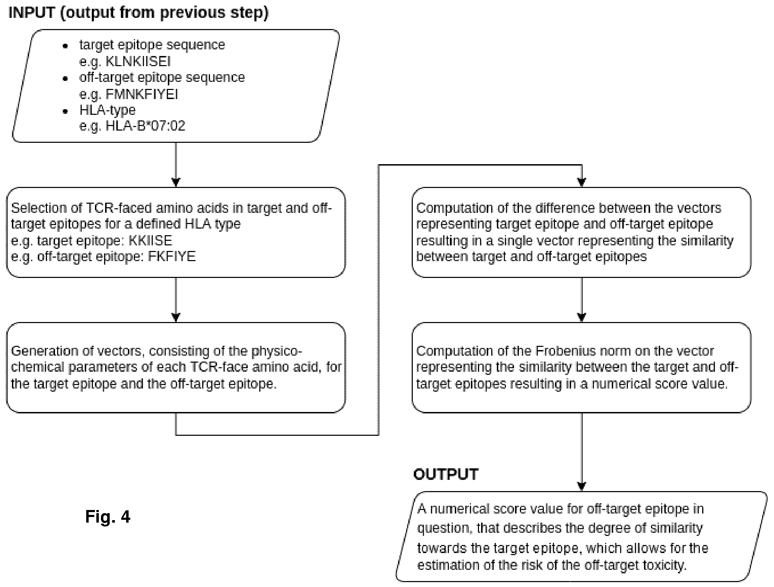

Fig. 4. shows a detailed pipeline relating to the step of ranking off-target epitopes;
Fig. 5-8 show histograms (A) presenting safety scores for putative off-target epitopes for 4 different cases. If available, expression values (mRNA and protein) of the genes that gave rise to the off-target epitope in said cases are presented in (B);
Fig. 9 shows an exemplary report at the end of the pipeline used in the method according to the invention;
Fig.10 shows an exemplary computer system for implementing the method according to the invention;
Fig. 11-14 show tables (1b, 2b, 3b and 4b) presenting exemplary mutations for 4 different cases;
Fig. 15-18 show tables (1c, 2c, 3c and 4c) presenting exemplary final outputs from the method pipeline for 4 different cases.

DETAILED DESCRIPTION

The method of predicting the occurrence of the off-target toxicity caused by significant similarity between a target epitope and off-target epitopes according to the invention can be useful for identifying cross reactivity in the early stage of development of TCR-therapies against specific target epitopes. Moreover, the method according to the invention can be of practical meaning also for development of immunological units containing antigen-recognition receptors.

While various embodiments of the invention are shown and described herein, such embodiments are provided by way of example only. Numerous variations, changes, and substitutions may occur to those skilled in the art without departing from the invention. It should be understood that various alternatives to the embodiments of the invention described herein may be employed.

Unless defined otherwise, all terms of art, notations and other technical and scientific terms or terminology used herein are intended to have the same meaning as is commonly understood by persons skilled in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

The term "binding affinity" or " binding specificity" when referring to a binding event, as used herein, generally refers to the degree to which a binding element or domain binds to a target protein or portion thereof, such as, for example, one or more peptides. For example, binding affinity may refer to the degree to which an antibody differentiates between two different antigens. Moreover, peptide binding affinity can be determined by the difference in the apparent Kd value for each array peptide in the absence of a competitor and in the presence of each serum and non-cognate peptide competitor.

The term "epitope" generally refers to a part of an antigen that an antibody/TCR will recognize and bind to.

The "off-target toxicity" describes the effects that can occur when a drug, or cell based therapeutic such as T-lymphocyte binds to targets (proteins or other molecules in the body) other than those for which the drug was meant to bind. This can lead to unexpected side effects that may be harmful.

The term "peptide", "peptides" or "polypeptides," as used herein, generally refers to polymer chains comprised of amino acid residue monomers which are joined together through amide bonds (peptide bonds). A peptide can be a chain of at least three amino acids, a protein, a recombinant protein, an antigen, an epitope, an enzyme, a receptor, or a structure analogue or combinations thereof. As used herein, the abbreviations for the L-enantiomeric amino acids that form a polypeptide are as follows: alanine (A, Ala); arginine (R, Arg); asparagine (N, Asn); aspartic acid (D, Asp); cysteine (C, Cys); glutamic acid (E, Glu); glutamine (Q, Gln); glycine (G, Gly); histidine (H, His); isoleucine (I, Ile); leucine (L, Leu); lysine (K, Lys); methionine (M, Met); phenylalanine (F, Phe); proline (P, Pro); serine (S, Ser); threonine (T, Thr); tryptophan (W, Trp); tyrosine (Y, Tyr); valine (V, Val).

The term T-cell as used herein, generally refers to cells in the adaptive immune system that attack invading pathogens and infected host cells depending on the T cell type activated. T cells can be distinguished from other lymphocytes by the presence of a T-cell receptor (TCR) on their cell surface.

The term TCR (T-cell receptor) as used herein, generally refers to a protein complex found on the surface of T cells that is responsible for recognizing fragments of antigen as peptides bound to major histocompatibility complex (MHC) molecules. Since the binding between TCR and antigen peptides has relatively low affinity and is degenerate, multiple TCRs can recognize the same antigen peptide and multiple antigen peptides may be recognized by the same TCR.

The term HLA as used herein, generally refers to the human leukocyte antigen system that is also known as the human version of the major histocompatibility complex (MHC) found in many animals. Its main role is in the presentation of peptides to the immune system. Based on HLA structure and function, there are two classes, Class I and Class II. The cell surface glycopeptide antigens of the HLA-A, HLA-B, and HLA-C series are called HLA Class I antigens. They are expressed on the surface of most nucleated cells of the body, thus enabling a cytotoxic T-cell to differentiate between a healthy and a tumor cell. The cell surface glycopeptide antigens HLA-DR, HLA-DQ, and HLA-DP are termed HLA Class II antigens. Since Class II antigens initiate a general immune response, they are present on immunologically active cells and not on all tissue.

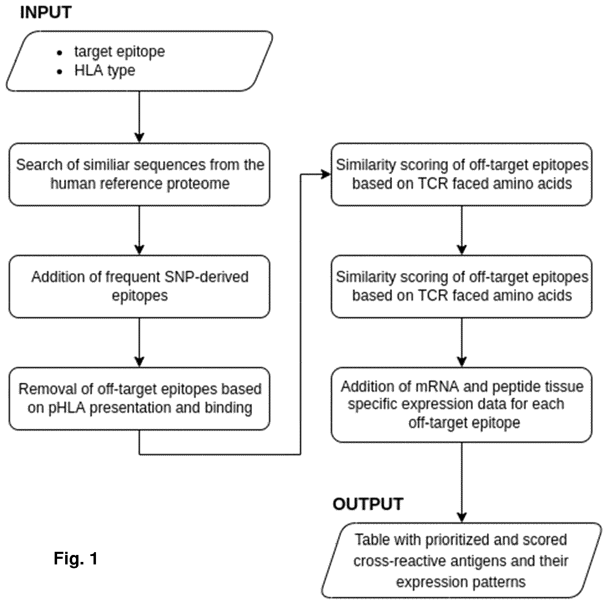

An off-target epitope (OTE), which causes toxicity can be identified in several consecutive steps of the method according to the invention as shown in Fig 1. All steps of the method according to the invention are computer implemented steps. The search may be conducted for target epitopes that are of human origin or of organisms which are commonly present in humans, such as viral or bacterial pathogens, or commensal bacteria.

The first step of the method consists of providing a target epitope for which toxicity should be checked as well as its corresponding HLA type (Human Leukocyte Antigens). In another embodiment not only one target epitope can be checked at once by the system according to the invention, which implements the method according to the invention, but several target epitopes and their corresponding HLA type can be inputted for further processing at once. However, all steps of the method are then performed by the system for each target epitope separately and only the results are outputted together. In said embodiment, an additional table summarizing all the results is generated, that is described below in the following part of the description.

The aim of the next step of the method is finding first all theoretically existing off-target epitopes and based on that all putative naturally existing off-target epitopes, namely those one that can be found in the reference proteome. It will be known to one skilled in the art, that in the context of the present invention, the term "reference proteome" is to be understood as a proteome that has been selected based on the target epitope for which toxicity should be checked. Said consecutive step of the method according to the invention, takes as an input a target epitope from 8 to 11 amino acids long and its corresponding HLA type. The first substep consists of identification of all epitopes that have at least 5 amino acids shared with the target epitope. This particular number was chosen because clinically relevant cases of cross reactivity showed that a minimum of 5 identical amino acids is needed for off-target toxicity to occur. Importantly, only off-target epitopes of the same length are taken into account as it is considered rare for epitopes of different lengths to bind to the same TCR as described in Ekeruche-Makinde J, Miles JJ, van den Berg HA, Skowera A, Cole DK, Dolton G, Schauenburg AJ, Tan MP, Pentier JM, Llewellyn-Lacey S, Miles KM, Bulek AM, Clement M, Williams T, Trimby A, Bailey M, Rizkallah P, Rossjohn J, Peakman M, Price DA, Burrows SR, Sewell AK, Wooldridge L. Peptide length determines the outcome of TCR/peptide-MHCI engagement. Blood. 2013 Feb 14;121(7):1112-23.

The present substep generates a large number of putative off-target epitopes, e.g. for a 8 amino acid target epitope there are 459360 possible similar epitopes according to a known formula:

$$\sum_{k=0,1\ldots n} \frac{N!}{(N!-k!)k!} * 20^k.$$

In practice, this substep is simplified and not full combinatorial generation of all possible combinations of sequences has to be performed e.g. for a 8 amino acid it would be enough to perform a search according to the formula

$$\frac{8!}{5!3!} * 1^3 = 56$$

as all possible position combinations are included in the screening assuming that a specific amino acid is replaced into any other amino acid (RegEx [A-Z]). At the end of this step a set of all artificially generated putative off-target epitopes is obtained. An advantage of this step is that it allows a high number of mismatches, as a consequence the cross-reactivity prediction method according to the invention becomes more robust and accurate

In the next substep, each of these artificially generated putative off-target epitopes is then checked whether it occurs in the reference proteome in order to select among them only putative off-target epitopes that are naturally existing within a given application context. Thus, said substep of the method involves selecting only those epitopes which are present in the reference proteome, in other words are natural. The data obtained from the screening consists of uniprot gene identification number, start position of the found peptide, end position of the identified peptide and amino acid sequence of the putative off-target epitope. Said data are stored for further processing. Specifically, some of them are used not in the consecutive step but later on in other steps. The reference used for the above-mentioned search can be found on the uniprot website (https://www.uniprot.org/proteomes/UP000005640), see also UniProt Consortium. UniProt: the universal protein knowledgebase in 2021, Nucleic Acids Res. 2021 Jan 8;49(D1):D480-D489, doi: 10.1093/nar/gkaa1100.

Then the method passes to a consecutive step of enlarging said set of all naturally existing putative off-target epitopes by addition of frequent single nucleotide polymorphisms (SNPs) in the previously identified putative off-target epitopes. One can say that by including frequent SNP-derived mutations the reference proteome is extended.

The person skilled in the art will appreciate that SNPs are the most frequent type of variants found in the human genome, see Shen LX, Basilion JP, Stanton VP Jr. Single-nucleotide polymorphisms can cause different structural folds of mRNA. Proc Natl Acad Sci U S A. 1999 Jul 6;96(14):7871-6, starting from as much as 7000 nonsynonymous mutations per individual, as mentioned in Kwak SH, Chae J, Choi S, Kim MJ, Choi M, Chae JH, Cho EH, Hwang TJ, Jang SS, Kim JI, Park KS, Bang YJ. Findings of a 1303 Korean whole-exome sequencing study. Exp Mol Med. 2017 Jul 14;49(7):e356. As such, SNPs can be a major source of novel off-target sites in TCR based therapies. Unfortunately, both rare and frequent SNPs are not included in the uniprot proteome reference sequences. The present invention tackles this problem in the step of enlarging said naturally existing set of putative off-target epitopes by peptides resulting from mutations. This particular step of the method according to the invention involves introducing frequent nonsynonymous mutations (occurring in more than 1% of at least one studied population) from gnomAD database - see Siwei Chen, Laurent C. Francioli, Julia K. Goodrich, Ryan L. Collins, Qingbo Wang, Jessica Alföldi, Nicholas A. Watts, Christopher Vittal, Laura D. Gauthier, et al. A genome-wide mutational constraint map quantified from variation in 76,156 human genomes. bioRxiv 2022.03.20.485034 (https://gnomad.broadinstitute.org/) based on putative off-target epitopes generated in the previous step.

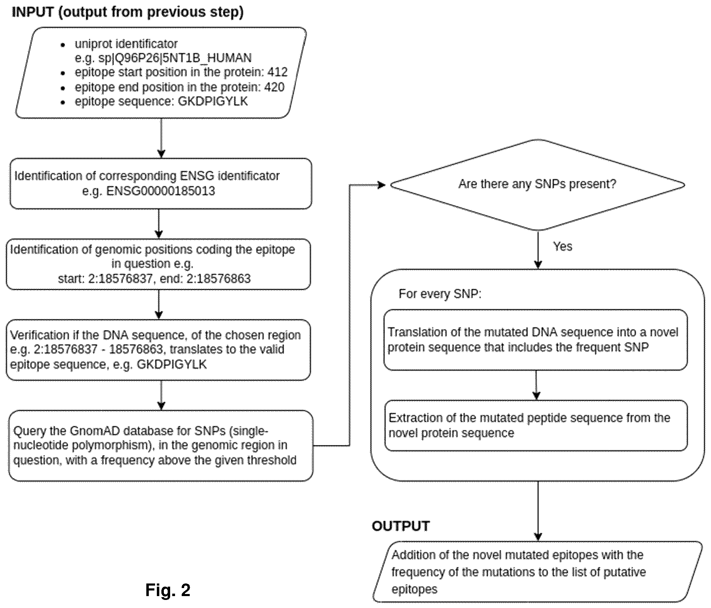

Each identified frequent SNPs is taken into account as an additional putative off-target epitope. For the off-target epitopes derived from SNPs their frequency in the largest human subpopulation is provided in final results. As shown in Fig. 2, this step comprises advantageously several substeps in which consecutive conversion of data relating to the set of all naturally existing putative off-target epitopes is performed.

First, in practice, preferably this step involves extracting uniprot identifiers from already saved names of peptides present in the human proteome for all putative off-target epitopes generated so far. Further, this step involves determining, which uniprot identifier corresponds to which ENSG identifier. In practice, advantageously a separate software module is used for converting said one identifier into another. Then, once the ENSG identifier is determined the genome region (reference hg19) which codes each putative off-target epitope is found using said ENSG identifier along with the start and end position of each off-target epitope. For example, for the putative off-target epitope which is GKDPIGYLK based on its name sp|Q96P26|5NT1B_HUMAN an ENSG identifier is received which is ENSG00000185013, while based on the start and end positions in the protein namely 412 and 420 its corresponding coding genome region is 2:18576837-18576863. Next, based on said found genome region relating to the respective off-target epitope, the gnomAD database is consulted for all frequent mutations in said region. If some mutations are found, then transcription of each frequent mutation in said genome region into an additional naturally existing off-target epitope is performed. The result of this particular step is an enlarged set of all naturally existing putative off-target epitopes by addition of epitopes resulting from mutations.

In another embodiment, as validation, an additional check is performed. It involves transcription of DNA of each specific genome region that has been found in the gnomAD database into an amino acid sequence in order to check whether a right genome region has been consulted in the gnomAD database.

It should be emphasized that because some genes might originate from a common ancestor gene several off-target epitopes having identical sequences can be found in the human proteome. This results in multiplication of similar cases, with a different gene indentificator on the final list of all naturally existing off-target epitopes at the end of the above mentioned step. Such multiplication of similar off-target epitopes on the final list can appear also because of different mathematical algorithms applied to artificial generation of all off-target epitopes being similar to the target epitope in the first step. At the end, the present step of enlarging said set of all naturally existing putative off-target epitopes through addition of epitopes derived from frequent SNPs further increases the number of off-target epitopes to be analyzed, which makes the algorithm more robust and patient specific.

The previous steps generate a high number of potential off-target epitopes (OTEs). However, only a small fraction of them will be bound and presented on a HLA class type considered for specific application. For this reason, the method according to the invention comprises another step which is extracting off-target epitopes that may be presented from said enlarged set of all naturally existing putative off-target epitopes. In order to limit the number of putative off-target epitopes to the relevant ones, a trained presentation model is used, however the person skilled in the art will appreciate that any presentation model can be used herein. This step involves selecting only a subset of off-target epitopes having surface HLA presentation probability for chosen HLA type above a presentation probability threshold value. Overall, one or two or more models can be used at once or separately to generate predictions for HLA type I and II in said step.

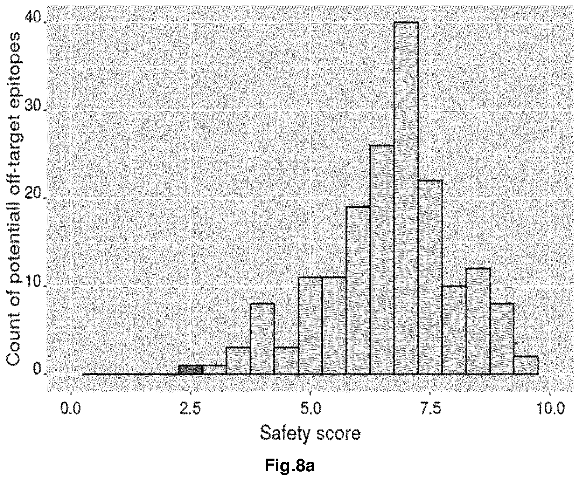

In practice, the presentation model is a computer implemented as a presentation model unit. The presentation model unit is one or more computer models, embodied in a computing system as discussed below with respect to Fig. 8, that receives peptide sequences associated with a set of MHC alleles and determines likelihoods that the peptide sequences will be presented by one or more of the set of associated MHC alleles.

The presentation model unit determines presentation likelihoods through one or more presentation models. Specifically, the presentation models generate likelihoods of whether given peptide sequences will be presented for a set of associated MHC alleles, and are generated based on presentation information stored in an associated database. The presentation information contains information on whether peptides bind to different types of MHC alleles such that those peptides are presented by MHC alleles, which in the models is determined depending on positions of amino acids in the peptide sequences.

As mentioned, any known presentation model can be used for that purpose, however the inventors used for the tests their own presentation model already mentioned in Mazzocco G, Niemiec I, Myronov A, Skoczylas P, Kaczmarczyk J, Sanecka-Duin A, Gruba K, Król P, Drwal M, Szczepanik M, Pyrc K, Stępniak P. "AI Aided Design of Epitope-Based Vaccine for the Induction of Cellular Immune Responses Against SARS-CoV-2" Front. Genet., 25 March 2021 Sec. Computational Genomics

The trained presentation model is based on machine learning methods, i.e. artificial neural networks, and trained on curated, publicly available datasets. In particular a curated dataset containing peptides presented by class I HLAs on the surface of host cells was extracted from publicly available databases (Abelin et al., Spectrometry Profiling of HLA-Associated Peptidomes in Mono-allelic Cells Enables More Accurate Epitope Prediction. Immunity. 2017 Feb 21 ;46(2):315-326; Di Marco et al., (2017). Unveiling the peptide motifs of HLA-C and HLA-G from naturally presented peptides and generation of binding prediction matrices. J. Immunol. 199 2639-265110.; Sarkizova et al., (2020). A large peptidome dataset improves HLA class I epitope prediction across most of the human population. Nat. Biotechnol. 38 199-209.).

The presentation of each peptide within the dataset was experimentally confirmed by mass-spectroscopy experiments that include at least proteasomal cleavage. All peptides were of human origin and were presented on the surfaces of monoallelic human cell lines. Synthetic negative data (non-presented peptides) were also prepared based on human proteome. The person skilled in the art will appreciate that the training data set can be prepared not only on mass-spectometry experiments.

Both peptide sequence and HLA type are taken into consideration as separate inputs. Regarding the training scheme, it involves bootstrapping and selecting 80% of positive examples during training with the remaining ones used for early stopping. Since the model is based on several networks an ensemble of the results is received. The used presentation model is pan-specific and can be used to generate predictions for any peptide and any canonical class I HLA (i.e., A, B, and C). It should be noted that the accuracy of the used method depends on the considered HLA type, as in the case of other machine learning methods for predicting pHLA properties.

The presentation model was implemented in Python 3.7 using the keras 2.4.3 package, which is a high-level API of TensorFlow. For our usage TensorFlow with GPU support was deployed, i.e., tensorflow-gpu 2.2.0. For GPU-based computations we used cudnn 7.6.5 and cudatoolkit 10.2.89 and a machine equipped with NVIDIA Tesla V100 GPU card with CUDA® 7.0 architecture, 640 Tensor Cores, 5,120 CUDA® Cores and 32 GB HBM2 GPU Memory. Additionally, scikit-learn, pandas, and numpy were used to perform standard machine learning tasks while images were produced using matplotlib and seaborn.

Since said trained model takes into consideration both peptide sequence and HLA type as separate inputs, the first substep includes inputting each off-target epitope within an enlarged set of naturally existing epitopes along with its corresponding HLA type in order to obtain as an output a probability of being presented on the cell surface. Said subset of off-target epitopes is then limited to only those which have surface HLA presentation probability for said HLA type above a presentation probability threshold value. In one embodiment, said presentation probability threshold value amounts to 50%. However advantageously said probability threshold value can be chosen as one among 40%, 41%, 42%, 43%, 44%, 45%, 46%, 47%, 48%, 49%, 50%, 51%, 52%, 53% etc.

In one embodiment, this step can further comprise a substep of selecting only a subset of off-target epitopes having binding affinity level below a binding affinity threshold value. In this optional substep, peptide - HLA binding is evaluated on the off-target epitopes using an open-source software: MHCflurry, see *"MHCflurry: Open-Source Class I MHC Binding Affinity Prediction"* Timothy J O'Donnell, Alex Rubinsteyn, Maria Bonsack, Angelika 8 Riemer, Uri Laserson, Jeff Hammerbacher". Said embedded software module outputs a numerical value in nM representative for binding affinity for each consecutive off-target epitope within the processed set. Only off-target epitopes that have an IC 50 value of binding affinity < 2000 nM proceed to the next steps. This additional substep can be performed before or after the use of the trained HLA presentation model. The aim of this substep is to further limit the set of putative off-target epitopes to high affinity epitopes because most of the presented epitopes data used for training the presentation model were high affinity. As a consequence the method according to the invention becomes more resistant for false positive cases.

The next and the key step of the method according to the present invention involves ranking the extracted subset of off-target epitopes, obtained in the previous steps, for its potential for toxicity by calculating a numerical score value for each off-target epitope that represents a degree of similarity between a target epitope and each off-target epitope. Said calculated score value, that can be called a safety score, is based on physico-chemical properties of the amino acids at predefined fixed positions interacting with antigen-recognition receptors. It will be appreciated by one skill in the art that the phrase "off-target epitope's potential for toxicity" refers to the toxicity caused by binding of e.g. an antigen-recognition receptor or an immune cell containing said receptor to an off-target epitope. Likewise, the term "antigen-recognition receptor" should be understood as a receptor that recognises and binds to a specific sequence presented on the cell surface e.g. a TCR or an antibody

In the first substep, the positions of TCR-faced residues, depending on the HLA type the epitope binds to, are established. It was shown that the assessment of the off-target epitopes' toxicity potential is influenced not only by the HLA binding affinity, but also by TCR binding. In epitopes, amino acids in different positions interact with either the HLA or the TCR. One of the advantages of the method according to the invention is a separate analysis of those two different interactions. By distinguishing amino acid positions facing MHC and TCR separately the method becomes more robust and the ranking of OTEs is more specific.

The scheme for predefining positions of amino acids in both the target epitope under analysis and putative off-target epitopes, which are crucial for TCR binding, is based on a literature search and results from the presentation model. As mentioned, the first substep relies on automated application of residues selection rules created as a result of deep analysis of scientific articles that directly specify and describe the so-called 'anchor residues' or 'TCR-faced residues' or 'TCR contact residues' that are known to interact preferentially with the HLA molecules. The anchor residues are specifically chosen for a specific HLA type.

The literature search consisted of identification of publications that performed Alanine-scan or X-scan on epitopes that were presented on a specific HLA type. Changes in amino acids in positions that negatively impacted EC50 of the T-cell (variable used to estimate affinity) were determined as TCR-faced residues. For example, in the following article describing an X-scan performed on an epitope presented on HLA A02:01 positions 3,4,7, were described as most sensitive for the TCR connection: Border EC, Sanderson JP, Weissensteiner T, Gerry AB, Pumphrey NJ. "Affinity-enhanced T-cell receptors for adoptive T-cell therapy targeting MAGE-A 10: strategy for selection of an optimal candidate. Oncoimmunology". If there was no Alanine-scan or X-scan data available for a certain HLA type, all non-HLA faced residues have been assumed to be TCR-faced.

Importantly, HLA-faced residues were selected from the IEDB database (https://www.iedb.org/). IEDB identifies these residues by generating a prediction matrix created by the Stabilized Matrix Method on mass-spectrometry data. The exact pipeline presented is available at the website https://help.iedb.org/hc/en-us/articles/114094147071-Motif-Display-Help For example, for HLA-B*07:02 type predefined positions being TCR residues saved in said database are 1,4,5,6,7 and 8.

In the next substep, amino acid sequences of the target epitope and off-target epitopes are limited by deleting amino acids in their sequences at fixed positions that are not established TCR-faced residues.

In the subsequent substep, the comparison between the target epitope and putative off-target epitopes is performed by focusing on the differences in physico-chemical properties of the TCR-facing residues. The inventors have proposed a new scheme for ranking off-target epitopes based on the spatial distance calculation between the most critical amino acids sequence in target epitope and each off-target epitope. Coordinates of said two objects in the space are defined by physico-chemical properties of the most critical amino acids sequences

To this end a matrix containing multiple types of amino acid properties for all known types of human amino acids was generated. The physico-chemical properties gathered in said matrix have been carefully selected and include at least:

1. amino acid secondary structure prediction beta-strand (see Blaber M, Zhang XJ, Matthews BW. Structural basis of amino acid alpha helix propensity. Science. 1993 Jun 11;260(5114):1637-40; Biou V, Gibrat JF, Levin JM, Robson B, Garnier J. Secondary structure prediction: combination of three different methods. Protein Eng. 1988 Sep;2(3):185-91);

2. aperiodic structure and alpha helix (see Blaber M, Zhang XJ, Matthews BW. Structural basis of amino acid alpha helix propensity. Science. 1993 Jun 11 ;260(5114):1637-40; Biou V, Gibrat JF, Levin JM, Robson B, Garnier J. Secondary structure prediction: combination of three different methods. Protein Eng. 1988 Sep;2(3):185-91);

3. sizes of atomic groups (sizes of radii and volume), (see Tsai J, Taylor R, Chothia C, Gerstein M. The packing density in proteins: standard radii and volumes. J Mol Biol. 1999 Jul 2;290(1):253-66);

4. relation of amino acid composition depending on protein localization (see Cedano J, Aloy P, Pérez-Pons JA, Querol E. Relation between amino acid composition and cellular location of proteins. J Mol Biol. 1997 Feb 28;266(3):594-600; Nakashima H, Nishikawa K. The amino acid composition is different between the cytoplasmic and extracellular sides in membrane proteins. FEBS Lett. 1992 Jun 1 ;303(2-3):141-6) and

5. inter-residue protein contact energies (see Miyazawa S, Jernigan RL. Self-consistent estimation of inter-residue protein contact energies based on an equilibrium mixture approximation of residues. Proteins. 1999 Jan 1;34(1):49-68).

As mentioned, for assessing the difference in physico-chemical properties of the TCR-facing residues a specific scheme with the use of the matrix of physico-chemical properties has been proposed. Advantageously, these particular physico-chemical properties were selected based on the paper by Saha I, Maulik U, Bandyopadhyay S, Plewczynski D. Fuzzy clustering of physicochemical and biochemical properties of amino acids. Amino Acids. 2012 Aug;43(2):583-94. doi: 10.1007/s00726-011-1106-9. In this work the authors grouped the amino acid indices obtained from the AAindex1 table in https://www.genome.jp/aaindex/ using a fuzzy clustering algorithm, identifying a representative AAindex for each cluster. This procedure allowed the selection of 8 non-redundant high quality amino acid indexes (i.e. HQI8) sufficient to describe multiple biologically relevant properties of amino acids such as isoelectric point, polarity, molecular weight, average accessible surface area, mutability, hydration potential, refractivity, optical activity and flexibility. Using HQI8 for protein encoding allows a compact representation of relevant amino acid properties. Advantageously, the matrix of physico-chemical properties is a combination of tables (if there is more than one model for a specific physico-chemical property) and each of these tables contains numerical values for every naturally occurring amino acid. Tables are created in Python as the dictionary data type.

Regarding details of the proposed comparison scheme, first, for both the target epitope and the off-target epitope under assessment, for each amino acid in the amino acid sequences limited to TCR-faced residues, advantageously, a vector of at least 5 values representing physico-chemical properties of said amino acid is generated based on data extracted from the embedded matrix mentioned above. As a result, since there are several TCR-faced residues, several vectors, each with at least 5 values representing physico-chemical properties of a predefined TCR-faced residue, are collected into one numerical value matrix data object for the target epitope and into one another numerical value matrix data object for the off-target epitope under assessment.

Next, both matrices are linearized into vectors, said resultant vectors being of the size $n=p_n * r_n$, where $p_n$ is the number of predefined residues and $r_n$ is the number of physico-chemical properties used for scoring. Then the distance between those two resultant vectors (one for the target epitope and one for the putative off-target epitopes) is calculated according to the following norm:

$$x = \sqrt{\sum_{i=1}^{n}(e_i - p_i)^2}$$

where $e_i$ and $p_i$ represent the *i-th* amino acids physico-chemical property from compared peptides (namely the target epitope and the off-target epitope) $e$ and $p$, and $n$ is equal to the length of the predefined sequence of TCR faced residues for both peptides multiplied by the number of physico-chemical properties used for the comparison and $x$ is the final score for each said off-target epitope. The person skilled in the art will appreciate that said length of the predefined sequence can relate to other types of antigen recognition receptors.

This final score x, namely a numerical score value for each off-target epitope, represents a degree of similarity between the target epitope and each said off-target epitope. This is the output of the step of ranking of each putative off-target epitope within the extracted subset based on physico-chemical properties of the amino acids at predefined fixed positions interacting with antigen-recognition receptors of off-target epitopes for its potential for toxicity.

The safety score can vary from 0 to 14 for a target and putative off-target epitope of the length of 8 amino acids.

However, scores up to 40 are possible for longer amino acid sequences. A score close to 0 means that the putative off-target epitope and the target epitope are almost identical from the perspective of pHLA:TCR interaction and it is highly probable for toxicity to occur. It means that the off-target under assessment has a high probability to cause toxicity.

If no off-target epitopes are found, or if the safety score shows that the off-target epitope is not expected to cause unwanted side effects (i.e. takes a value above 3), the target epitope may be used in treating a disease or condition which is ameliorated by administration of the binding peptide, e.g., an antibody, directed against such a target. The person skilled in the art will appreciate that an arbitrary threshold value can be established, different than 3, for example 3.5, 4, 4.5, 5, 5.5, 6, 6.6 etc.

Methods of treatment include but are not limited to immunotherapies; for example, administration to a patient of modified T cells (adoptive therapy), such as those transduced with affinity enhanced T cell receptors; administration of monoclonal antibodies or monoclonal antibody fragments, especially TCR-like antibodies; administration of novel bi-specific immunotherapeutic agents such as ImmTACs.

In some situations, the binding peptide may not be administered *in vivo* because of the cross reaction that this can cause (i.e. safety score takes a value below 3). Then, the target epitope may be redesigned and again verified by the method of the present invention, whether any cross reactivity occurs.

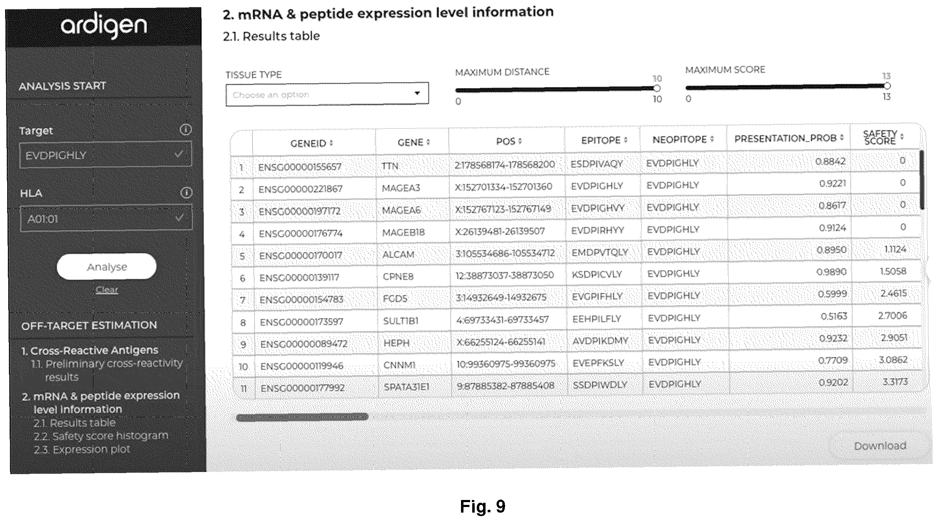

In the final step, once one or more off-target epitopes have been identified, mRNA expression in TPMs (GTEx) (see GTEx Consortium. The Genotype-Tissue Expression (GTEx) project. Nat Genet. 2013 Jun;45(6):580-5) and protein expression level (Human Protein Atlas) (see Uhlén M, et al. Tissue-based map of the human proteome. Science. 2015 Jan 23;347(6220):1260419) of the putative off-target epitopes are added in an attempt to identify tissues sensitive to off-target toxicity. If expression of the off-target epitope in non-embryonic tissue is non-existent or limited, the target epitope may be considered suitable for medical treatment *in vivo.* In cases, where expression of the off-target epitope in non-embryonic tissue is widespread or is present in critical tissues, such as heart cells, such a target epitope should not be further considered in work on the design of binding proteins directed against such epitopes. The output of this step is one or more reports with information on expression levels (transcripts, proteins), prioritized list of all off-target epitopes etc. An exemplary report can be seen in Fig. 9.

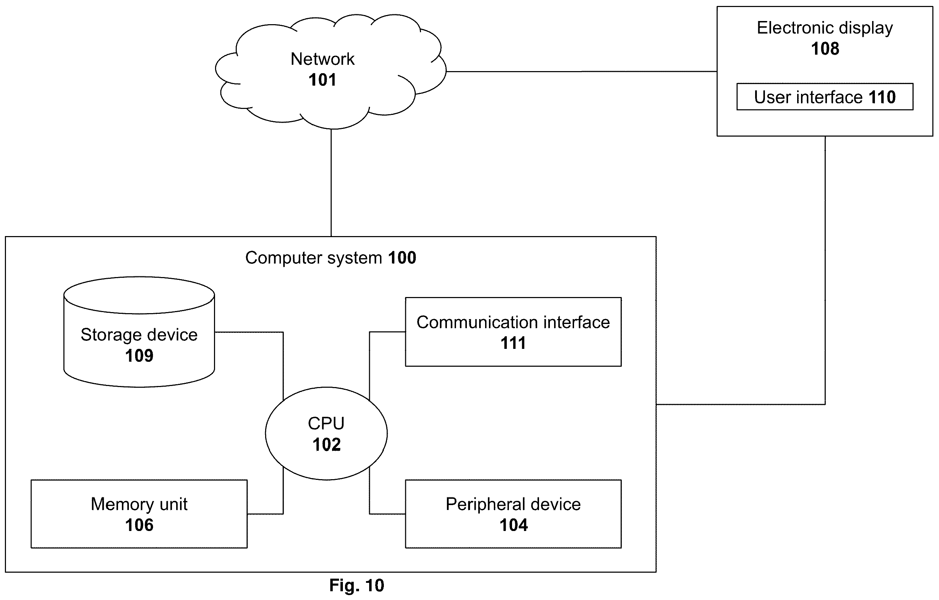

An exemplary system configured to perform the method according to the invention is shown in Fig. 10. The system comprises an example computer system 100 for implementing the entities shown in Fig. 1. The computer system 100 includes a central processing unit (CPU, also "processor" and "computer processor" herein) 102, which can be a single core or multi core processor, either through sequential processing or parallel processing. The computer system 100 also includes a memory unit or device 106 (e.g., random-access memory, read-only memory, flash memory), a storage unit or device 109 (e.g., hard disk), a communication interface 111 (e.g., network adapter) for communicating with one or more other systems, and peripheral devices, either external or internal or both, such as a printer, monitor, USB drive and/or CD-ROM drive. It also comprises the touch-screen interface, a mouse, track ball, or other type of pointing device, a keyboard, or some combination thereof, and is used to input data into the computer system 100. The memory 106, storage unit 109, communication interface 111 and peripheral devices 104 are in communication with the CPU 102 through a communication bus (solid lines), such as a motherboard. The storage unit 109 can be a data storage unit (or data repository) for storing data. The computer system 100 can be operatively coupled to a computer network ("network") 101 with the aid of the communication interface 111. The network 101 can be the Internet, an internet and/or extranet, or an intranet and/or extranet that is in communication with the Internet. The network 101 in some cases is a telecommunication and/or data network. The network 101 can include one or more computer servers, which can enable a peer-to-peer network that supports distributed computing. The network 101, in some cases with the aid of the computer system 100, can implement a client-server structure, which may enable devices coupled to the computer system 100 to behave as a client or a server. Other embodiments of the computer system 100 have different architectures.

The storage device 109 is a non-transitory computer-readable storage medium such as a hard drive, compact disk read-only memory (CD-ROM), DVD, or a solid-state memory device. The memory 106 holds instructions and data used by the processor (CPU) 102.

The computer system 100 can include or be in communication with an electronic display 108 that comprises a user interface (UI) 110. Examples of UI's include, without limitation, a graphical user interface (GUI) and web-based user interface. The graphics adapter (not shown) displays images and other information on the display 108. The network adapter (communication interface) 111 couples the computer system 100 to one or more computer networks. The network 101 can be the Internet, an internet and/or extranet, or an intranet and/or extranet that is in communication with the Internet. The network 101 in some cases is a telecommunication and/or data network. The network 101 can include one or more computer servers, which can enable a peer-to-peer network that supports distributed computing. The network, in some cases with the aid of the computer system, can implement a client-server structure, which may enable devices coupled to the computer system to behave as a client or a server.

The computer system 100 is adapted to execute computer program modules for providing functionality described herein. As used herein, the term "module" refers to computer program logic used to provide the specified functionality. Thus, a module can be implemented in hardware, firmware, and/or software. In one embodiment, program modules are stored on the storage device 109, loaded into the memory 106, and executed by the processor 102.

Types of computer systems 100 used by the entities of Fig. 1 can vary depending upon the embodiment and the processing power required by the entity. For example, the presentation model unit can run in a single computer 100 or multiple computers 100 communicating with each other through a network such as in a server farm. The computer system 100 can regulate various aspects of the present disclosure, such as, for example, inputting amino acid position information, transferring imputed information into datasets, and generating a trained algorithm with the datasets. The computer system 100 can be an user electronic device or a remote computer system. The electronic device can be a mobile electronic device. The computer system 100 can lack some of the components described above, such as graphics adapters, and displays 108.

Provided herein is also a non-transitory computer readable medium comprising machine-executable code that, upon execution by one or more computer processors, implements a method for predicting the occurrence of the off-target toxicity caused by similarity between a target epitope and putative off-target epitopes.

The code can be pre-compiled and configured for use with a machine having a processor adapted to execute the code, or it can be compiled during runtime. The code can be supplied in a programming language that can be selected to enable the code to execute in a pre-compiled or as-compiled fashion.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as the main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

In reference to Fig.1, the whole pipeline was implemented using Python 3.7 (scikit-learn, pandas, and numpy) (see Scikit-learn: Machine Learning in Python, Pedregosa et al., JMLR 12, pp. 2825-2830, 2011; Reback, J., McKinney, W., et al. Pandas-Dev/Pandas: Pandas 1.2.2, Zenodo [code], https://doi.org/10.5281/zenodo.4524629, 2021; Harris, C.R., Millman, K.J., van der Walt, S.J. et al. Array programming with NumPy. Nature 585, 357-362 (2020). DOI: 10.1038/s41586-020-2649-2) and R v4.0.2 (dplyr, ggplot2 and BSgenome) (see Wickham H, François R, Henry L, Müller K (2022). dplyr: A Grammar of Data Manipulation; Wickham H (2016). ggplot2: Elegant Graphics for Data Analysis. Springer-Verlag New York. ISBN 978-3-319-24277-4; Pagès H (2022). BSgenome: Software infrastructure for efficient representation of full genomes and their SNPs). The webserver was developed with Django, a high level Python web framework and can be accessed upon request (see Django Software Foundation. Django [Internet]. 2019. Available from: https://djangoproject.com).

The inventors selected for testing the method according to the invention three groups of peptides, where two of them (TAA epitopes, Virus epitopes), were acquired from IEDB (https://www.iedb.org/) (see Vita R, Mahajan S, Overton JA, Dhanda SK, Martini S, Cantrell JR, Wheeler DK, Sette A, Peters B. The Immune Epitope Database (IEDB): 2018 update. Nucleic Acids Res. 2018 Oct 24), while the last one containing epitopes generated from frameshift mutations was obtained from a library of Neo Open Reading Frame peptides (see Koster J, Plasterk RHA. A library of Neo Open Reading Frame peptides (NOPs) as a sustainable resource of common neoantigens in up to 50% of cancer patients. Sci Rep. 2019 Apr 29;9(1):6577). The IEDB dataset for TAA epitopes included the following tags 'human HLA-A*02:01' or 'human HLA-A*01:01', 'Linear Epitopes', 'Positive Assays only', 'T cells Assays', 'MHC ligand Assays', 'No B-cell assays', 'Host: Homo Sapiens (Human)', 'Disease: Cancer'. Additionally for the virus epitopes we added the tag 'Organism: DNA virus'. We randomly selected 148 epitopes for both TAA and virus datasets (supplementary table 1).

Furthermore, a frameshift database for tests has been developed. It is composed of 16 epitopes (out of 3003 epitopes) identified by our model as being presented by HLA on the cell surface. Importantly, 16 TAA presented on HLA-A*02:01 were randomly selected to compare TAA vs frameshift epitopes on equally abundant groups.

EXAMPLES

The present invention will now be presented in use with reference to the following examples which are all based on 4 clinically validated cases of TCRs targeting tumor associated antigens.

EXAMPLE 1

The first target epitope taken as an input was KVAELVHFL. This epitope has been found as potentially useful for treating melanoma and metastatic cancer. Its corresponding HLA-A type is A02:01. In the step of identifying a set of all naturally existing putative off-target epitopes having the same length as the predefined length of said target epitope and having not more than 4 mismatches, a set of 1351 epitopes was obtained.

A fragmentary table containing the output of said step (see Table 1a) is presented below. It contains only 20 consecutive lines selected from a total of 1351.

Table 1a

| Id | start | end | Peptide2 |
|---|---|---|---|
| sp\|P43356\|MAGA2_HUMAN | 112 | 120 | KMVELVHFL |
| sp\|P43357\|MAGA3_HUMAN | 112 | 120 | KVAELVHFL |
| sp\|P43358\|MAGA4_HUMAN | 113 | 121 | KVDELAHFL |
| sp\|P43359\|MAGA5_HUMAN | 112 | 120 | KVADLIHFL |
| sp\|P43360\|MAGA6_HUMAN | 112 | 120 | KVAKLVHFL |
| sp\|P43361\|MAGA8__HUMAN | 115 | 123 | KVAELVRFL |
| sp\|P43362\|MAGA9_HUMAN | 111 | 119 | KVAELVHFL |
| sp\|P43363\|MAGAA_HUMAN | 137 | 145 | KVTDLVQFL |
| sp\|P43364\|MAGAB_HUMAN | 225 | 233 | KIIDLVHLL |
| sp\|P43365\|MAGAC_HUMAN | 112 | 120 | KMAELVHFL |
| sp\|O15479\|MAGB2_HUMAN | 114 | 122 | KSGSLVQFL |
| sp\|O15480\|MAGB3_HUMAN | 114 | 122 | KTNMLVQFL |
| sp\|O15481\|MAGB4_HUMAN | 112 | 120 | KTKMLVQFL |
| sp\|Q9BZ81\|MAGB5_HUMAN | 43 | 51 | KVGLLEQFL |
| sp\|Q96LZ2\|MAGBA_HUMAN | 114 | 122 | KVIILVHYL |
| sp\|A2A368\|MAGBG_HUMAN | 116 | 124 | KVAFLVNFM |
| sp\|A8MXT2\|MAGBH_HUMAN | 112 | 120 | KTGELVQFL |
| sp\|A8MXT2\|MAGBH_HUMAN | 285 | 293 | KVLEFVAKL |
| sp\|Q96M61\|MAGBI_HUMAN | 110 | 118 | KVVSLVHFL |
| sp\|O60732\|MAGC1_HUMAN | 911 | 919 | KVDELARFL |

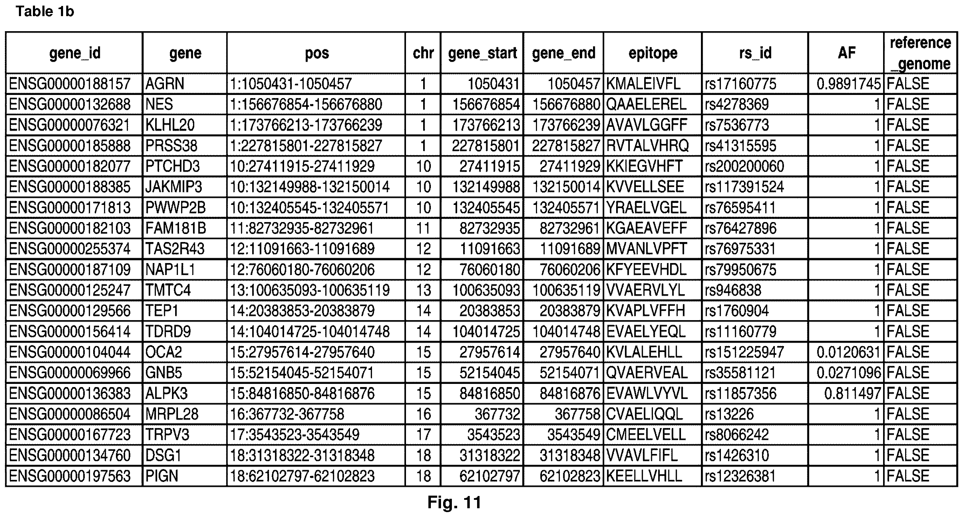

Then an additional set of 55 epitopes was found in the step of enlarging said set of all naturally existing putative off-target epitopes through identification of additional naturally existing off-target epitopes by applying Single nucleotide Polymorphisms mutation analysis. Only 20 exemplary mutations that have been found in this step are presented in the Table 1b shown in Fig. 11.

Next, the whole enlarged set of off-target epitopes was inputted into a binding affinity prediction model. This step was performed with the use of the commonly available MHCflurry open-source software module (https://github.com/openvax/mhcflurry). Once the binding affinity was obtained for the whole inputted set of off-target epitopes, only high affinity epitopes (IC 50 value of affinity <2000 nM) are chosen for the next step.

Next, in the step of selecting only a subset of off-target epitopes having surface HLA presentation probability for said HLA type above a presentation probability threshold value, the previously obtained set of off-target epitopes was inputted into the presentation model, and only 300 epitopes were found as having probability of surface presentation above 50%.

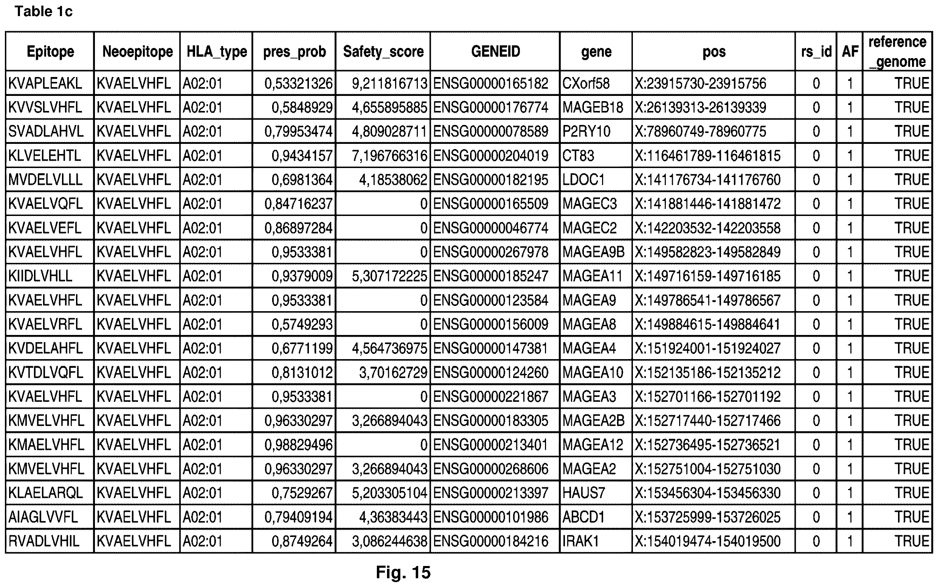

During the next step of ranking said extracted subset of off-target epitopes a safety score level for each off-target epitope is obtained. Exemplary outputs for said step are presented in the Table 1c shown in Fig. 15. Again only 20 consecutive lines selected from a total of 300 are presented.

As it can be seen also a known toxic epitope KMAELVHFL associated with the gene MAGEA12 has been identified and ranked with the safety score largely below 3.

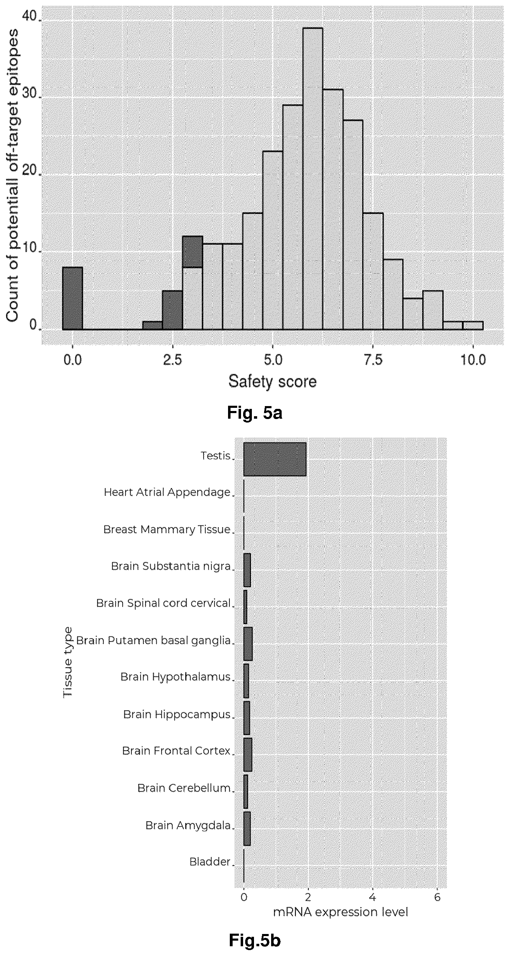

In the final step there is presented mRNA expression in TPMs and protein expression level of the putative off-target epitopes in a figure format. Furthermore, a histogram with the distribution of all the safety scores is generated. Fig. 5A, shows the exemplary outputs for said step.

EXAMPLE 2

The second target epitope taken as an input was EVDPIGHLY. This epitope has been found as potentially useful for curing myeloma and melanoma Its corresponding HLA type is HLA-A*01:01. In the step of identifying a set of all naturally existing putative off-target epitopes having the same length as the predefined length of said target epitope and having not more than 4 mismatches, a set of 464 epitopes were obtained.

A fragmentary table containing the output of said step (see Table 2a) is presented below. It contains only 20 consecutive lines selected from a total of 464.

Table 2a

| Id | start | end | Peptide2 |
|---|---|---|---|
| sp\|O15119\|TBX3_HUMAN | 100 | 108 | EDDPKVHLE |
| sp\|O95947\|TBX6_HUMAN | 201 | 209 | TLDPHGHLI |
| sp\|Q9UKZ4\|TEN1_HUMAN | 1501 | 1509 | AVSPDGTLY |
| sp\|Q9UKZ4\|TEN1_HUMAN | 1654 | 1662 | EYDPEGHLT |
| sp\|Q9UP52\|TFR2_HUMAN | 263 | 271 | GVDPVGRLL |
| sp\|Q7Z6K1\|THAP5_HUMAN | 316 | 324 | EVLQIEHSY |
| sp\|Q13009\|TIAM1_HUMAN | 866 | 874 | EEDGIRRLY |
| sp\|Q8IVF5-5\|TIAM2_HUMAN | 811 | 819 | EVDELLHIY |
| sp\|Q86X45\|TILB_HUMAN | 417 | 425 | EVDPSKHSF |
| sp\|Q8WZ42\|TITIN_HUMAN | 24337 | 24345 | ESDPIVAQY |
| sp\|Q8WZ42-6\|TITIN_HUMAN | 3816 | 3824 | ESLPKDHLY |
| sp\|Q9Y4G6\|TLN2_HUMAN | 1707 | 1715 | VVQEIGHLI |
| sp\|Q15399\|TLR1_HUMAN | 155 | 163 | SVLPIAHLN |
| sp\|O00206\|TLR4_HUMAN | 142 | 150 | ENFPIGHLK |
| sp\|Q9UK28\|TM59L_HUMAN | 220 | 228 | HVDPVGPLD |
| sp\|Q8IU80\|TMPS6_HUMAN | 196 | 204 | EVDPEGLVI |
| sp\|O43557\|TNF14_HUMAN | 91 | 99 | EVNPAAHLT |
| sp\|Q6ICL3\|TNG2_HUMAN | 108 | 116 | KVSMEGHLY |
| sp\|Q9UPQ9\|TNR6B_HUMAN | 1570 | 1578 | SPDPIGHNP |
| sp\|Q9Y2L5\|TPPC8_HUMAN | 851 | 859 | TVDGIGALP |

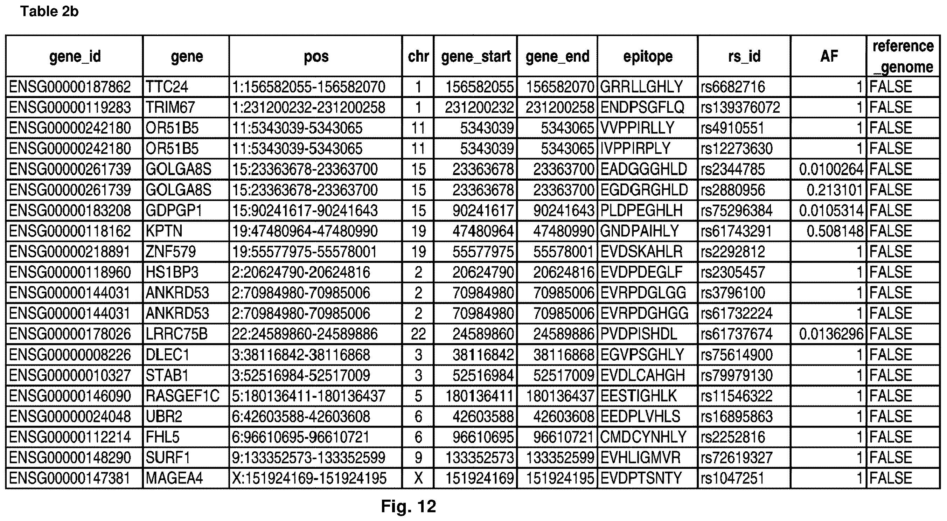

Then an additional set of 20 epitopes was found in the step of enlarging said set of all naturally existing putative off-target epitopes through identification of additional naturally existing off-target epitopes by applying Single nucleotide Polymorphisms mutation analysis. Only 20 exemplary mutations that have been found in this step are presented in the Table 2b shown in Fig. 12.

Next, the whole enlarged set of off-target epitopes was inputted into a binding affinity prediction model. This step was performed with the use of the commonly available MHCflurry open-source software module (https://github.com/openvax/mhcflurry). Once the binding affinity was obtained for the whole inputted set of off-target epitopes, only high affinity epitopes (affinity <2000 nM) are chosen for the next step.

Next, in the step of selecting only a subset of off-target epitopes having surface HLA presentation probability for said HLA type above a presentation probability threshold value, the previously obtained set of off-target epitopes was inputted into the presentation model, and only 85 epitopes were found as having probability of surface presentation above 50%.

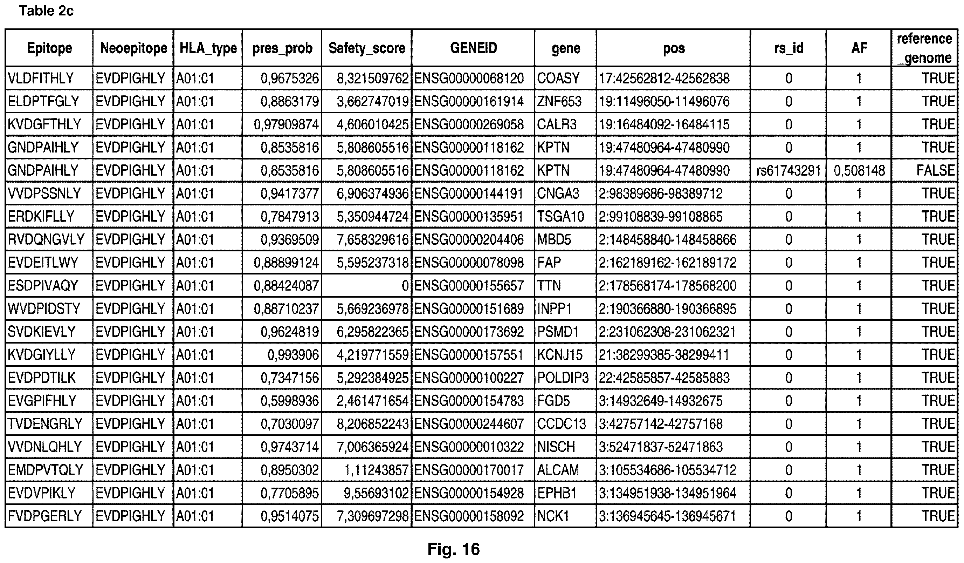

During the next step of ranking said extracted subset of off-target epitopes by calculating safety score level for each off-target epitope, there is obtained a matrix which contains numerical value. Exemplary outputs for said step are presented in the Table 2c shown in Fig. 16. Again only 20 consecutive lines selected from a total of 85 are presented.

As it can be seen also a known toxic epitope ESDPIVAQY associated with the gene TTN has been identified and ranked with the safety score below 3.

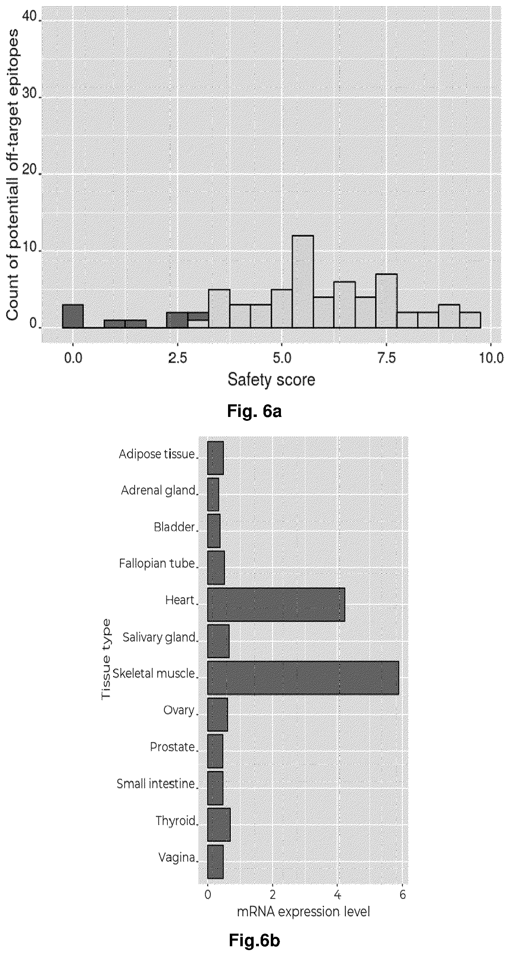

In the final step there is presented mRNA expression in TPMs and protein expression level of the putative off-target epitopes in a figure format. Furthermore, a histogram with the distribution of all the safety scores is generated. Fig. 6A, shows the exemplary outputs for said step.

EXAMPLE 3

The first target epitope taken as an input was FMNKFIYEI. This epitope has been found as potentially useful for curing liver cancer Its corresponding HLA type is HLA-A*02:01 In the step of identifying a set of all naturally existing putative off-target epitopes having the same length as the predefined length of said target epitope and having not more than 4 mismatches, a set of 233 epitopes was obtained.

A fragmentary table containing the output of said step (see Table 3a) is presented below. It contains only 20 consecutive lines selected from a total of 233.

Table 3a

| Id | start | end | Peptide2 |
|---|---|---|---|
| sp\|Q8NCN5\|PDPR_HUMAN | 834 | 842 | FINRGEYEI |
| sp\|Q9UMS5\|PHTF1_HUMAN | 726 | 734 | TMNPLIYNI |
| sp\|Q92508\|PIEZ1_HUMAN | 990 | 998 | FFYKFGLEI |
| sp\|Q8TEQ8\|PIGO_HUMAN | 1044 | 1052 | FAPKFIFEA |
| sp\|O00562\|PITM1_HUMAN | 103 | 111 | FVEKFSIEI |
| sp\|P41247\|PLPL4_HUMAN | 65 | 73 | FTYKFAEEI |
| sp\|Q8N8Z3\|PRR26_HUMAN | 67 | 75 | ADNIFIYES |
| sp\|Q5H9M0\|PWP3B_HUMAN | 678 | 686 | FDAKIIYEK |
| sp\|Q86YV0\|RASL3_HUMAN | 663 | 671 | FMNSFLEEH |
| sp\|Q9Y2P8\|RCL1_HUMAN | 215 | 223 | ILNKFIPDI |
| sp\|A1A4S6\|RHG10_HUMAN | 31 | 39 | RTNKFIKEL |
| sp\|Q9UNA1\|RHG26_HUMAN | 31 | 39 | KTNKFIKEL |
| sp\|A6NI28\|RHG42_HUMAN | 31 | 39 | RTNKFIKEL |
| sp\|Q15669\|RHOH_HUMAN | 94 | 102 | LKNKWIGEI |
| sp\|Q9BVS4\|RIOK2_HUMAN | 273 | 281 | FMKRFSYES |
| sp\|Q13546\|RIPK1_HUMAN | 222 | 230 | FANKEPYEN |
| sp\|P39023\|RL3_HUMAN | 279 | 287 | EINKKIYKI |
| sp\|Q9NWF9\|RN216_HUMAN | 298 | 306 | VANGFIEEI |
| sp\|Q9HAU8\|RNPL1_HUMAN | 436 | 444 | LMNLFTYEK |
| sp\|P56715\|RP1_HUMAN | 1519 | 1527 | RMNGIIYEI |

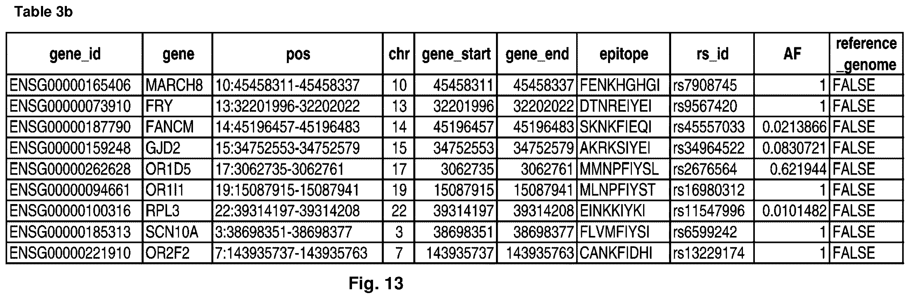

Then an additional set of 9 epitopes was found in the step of enlarging said set of all naturally existing putative off-target epitopes through identification of additional naturally existing off-target epitopes by applying Single nucleotide Polymorphisms mutation analysis. All 9 additional epitopes that have been found in this step are presented in the Table 3b shown in Fig. 13.

Next, the whole enlarged set of off-target epitopes was inputted into a binding affinity prediction model. This step was performed with the use of the commonly available MHCflurry open-source software module (https://github.com/openvax/mhcflurry). Once the binding affinity was obtained for the whole inputted set of off-target epitopes, only high affinity epitopes (IC 50 value of affinity <2000 nM) are chosen for the next step

Next, in the step of selecting only a subset of off-target epitopes having surface HLA presentation probability for said HLA type above a presentation probability threshold value, the previously obtained set of off-target epitopes was inputted into the presentation model, and only 39 epitopes were found as having probability of surface presentation above 50%.

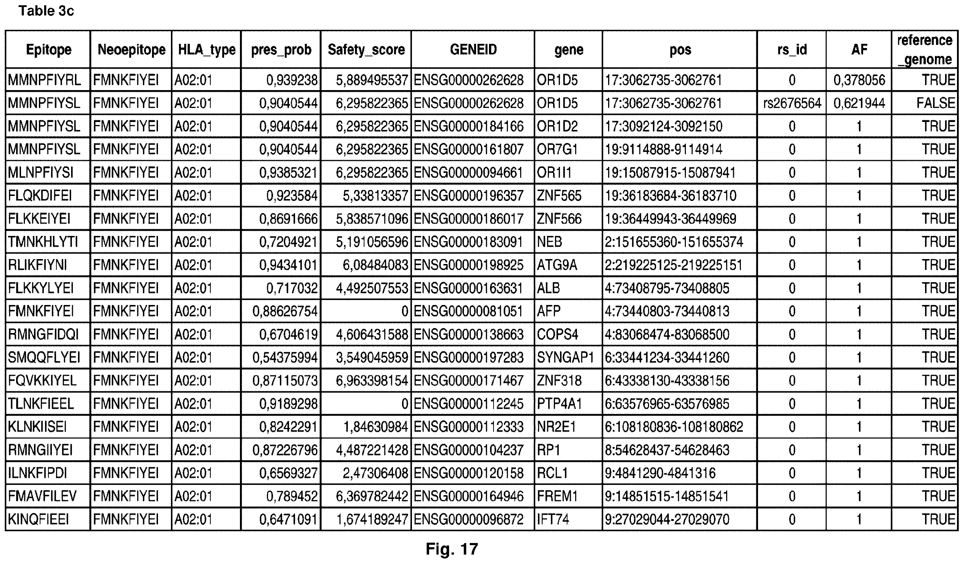

During the next step of ranking said extracted subset of off-target epitopes by calculating safety score level for each off-target epitope, there is obtained a matrix which contains numerical value. Exemplary outputs for said step are presented in the Table 3c shown in Fig. 17.

Again only 20 consecutive lines selected from a total of 39 are presented.

As it can be seen also a clinically known epitope ILNKFIPDI associated with the gene RLC1 has been identified and ranked with the safety score below 3.

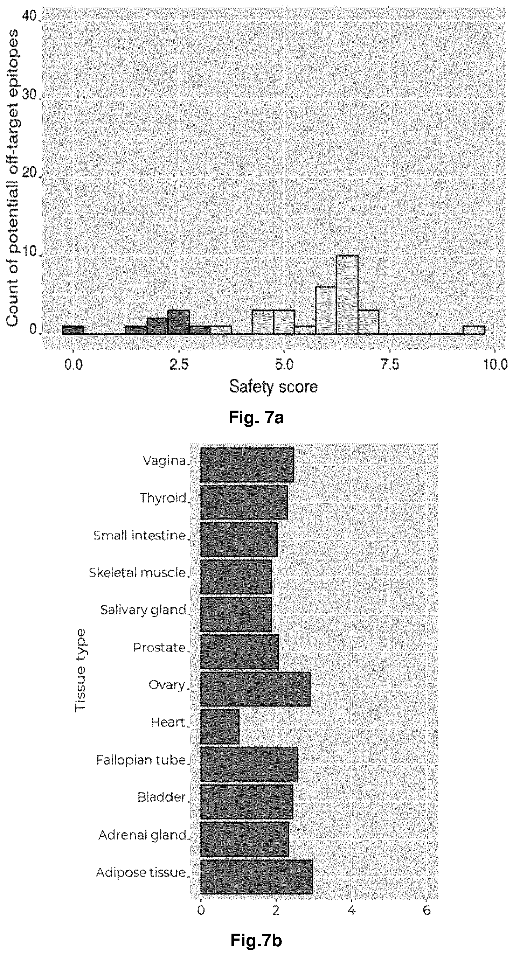

In the final step there is presented mRNA expression in TPMs and protein expression level of the putative off-target epitopes in a figure format. Furthermore, a histogram with the distribution of all the safety scores is generated. Fig. 7A, shows the exemplary outputs for said step.

EXAMPLE 4

The first target epitope taken as an input was SLLMWITOV. This epitope has been found as potentially useful for curing myeloma Its corresponding HLA type is HLA-A*02:01 In the step of identifying a set of all naturally existing putative off-target epitopes having the same length as the predefined length of said target epitope and having not more than 4 mismatches, a set of 588 epitopes was obtained.

A fragmentary table containing the output of said step (see Table 4a) is presented below. It contains only 20 consecutive lines selected from a total of 588.

Table 4a

| Id | start | end | Peptide2 |
|---|---|---|---|
| sp\|O75449\|KTNA1_HUMAN | 2 | 10 | SLLMISENV |
| sp\|P06312\|KV401_HUMAN | 10 | 18 | SLLLWISGA |
| sp\|P07942\|LAMB1_HUMAN | 1770 | 1778 | SLLKDISOK |
| sp\|Q6UWM7\|LCTL_HUMAN | 549 | 557 | SLCVLITAV |
| sp\|P11150\|LIPC_HUMAN | 289 | 297 | SLLHAGTQS |
| sp\|Q8N485\|LIX1_HUMAN | 7 | 15 | SLRHIIAQV |
| sp\|Q96S06\|LMF1_HUMAN | 309 | 317 | SFLNWLTMV |
| sp\|Q9BU23\|LMF2_HUMAN | 382 | 390 | SALWRWTQV |
| sp\|P16050\|LOX15_HUMAN | 587 | 595 | SLQMSITWQ |
| sp\|P50851\|LRBA_HUMAN | 657 | 665 | FLLMFIKQL |
| sp\|Q6P9F7\|LRC8B_HUMAN | 547 | 555 | SSLSRIPQV |
| sp\|Q9NZR2\|LRP1B_HUMAN | 1300 | 1308 | SLLYWTDVV |
| sp\|Q6UXK5\|LRRN1_HUMAN | 68 | 76 | SNLSSDTQV |
| sp\|Q6UWE0\|LRSM1_HUMAN | 367 | 375 | EOLMSITOE |
| sp\|Q8NDX9\|LY65B_HUMAN | 53 | 61 | SLCMVITIY |
| sp\|Q5VWZ2\|LYPL1_HUMAN | 36 | 44 | GLRMWIKOV |
| sp\|Q99698\|LYST_HUMAN | 2605 | 2613 | SLLMKMRSV |
| sp\|O95382\|M3K6_HUMAN | 555 | 563 | SLLEPETQD |
| sp\|Q96JQ5\|M4A4A_HUMAN | 133 | 141 | SLGMNITSS |
| sp\|Q96JQ5\|M4A4A_HUMAN | 77 | 85 | SLSMGITMM |

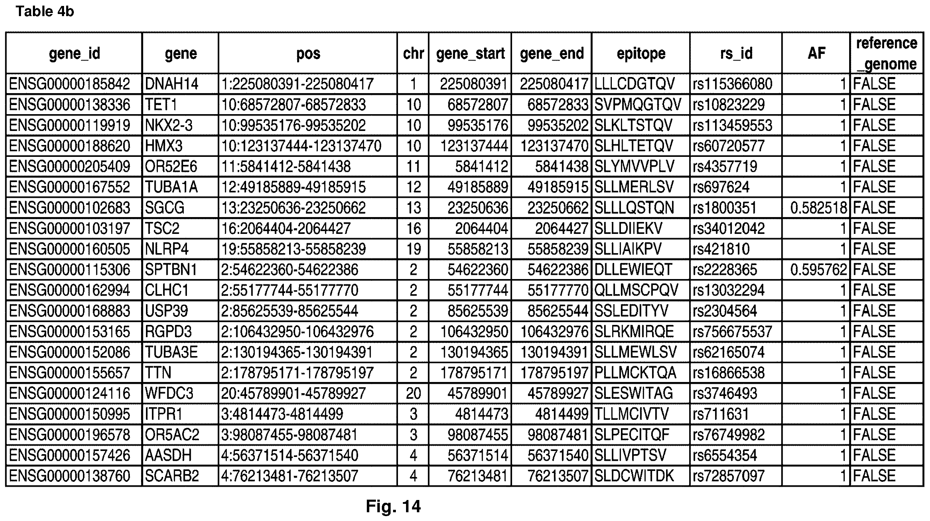

Then an additional set of 27 epitopes was found in the step of enlarging said set of all naturally existing putative off-target epitopes through identification of additional naturally existing off-target epitopes by applying Single nucleotide Polymorphisms mutation analysis. Only 20 exemplary mutations that have been found in this step are presented in the Table 4b shown in Fig. 14.

Next, the whole enlarged set of off-target epitopes was inputted into a binding affinity prediction model. This step was performed with the use of the commonly available MHCflurry open-source software module (https://github.com/openvax/mhcflurry). Once the binding affinity was obtained for the whole inputted set of off-target epitopes, only high affinity epitopes (IC 50 value of affinity <2000 nM) are chosen for the next step.

Next, in the step of selecting only a subset of off-target epitopes having surface HLA presentation probability for said HLA type above a presentation probability threshold value, the previously obtained set of off-target epitopes was inputted into the presentation model, and only 209 epitopes were found as having probability of surface presentation above 50%.

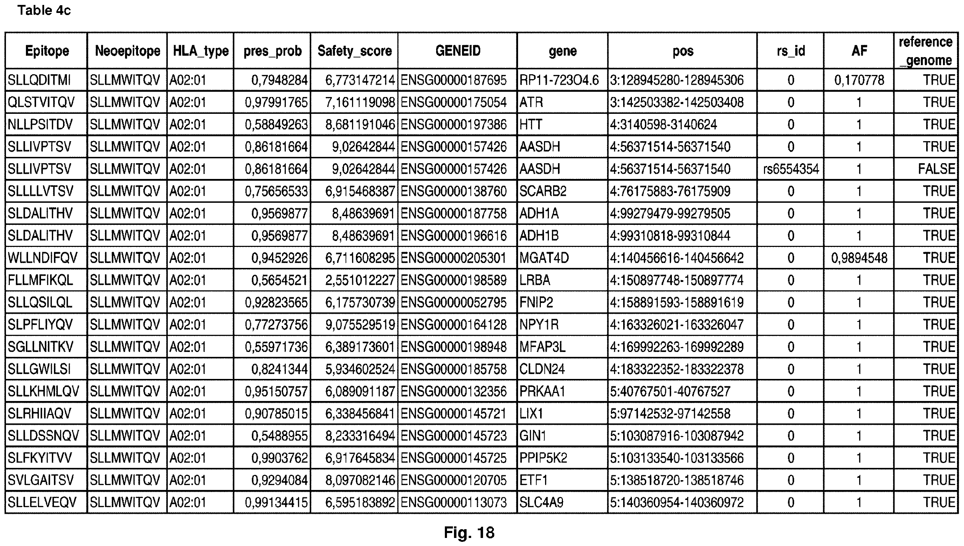

During the next step of ranking said extracted subset of off-target epitopes by calculating safety score level for each off-target epitope, there is obtained a matrix which contains numerical value. Exemplary outputs for said step are presented in the Table 4c shown in Fig. 18.

Again only 20 consecutive lines selected from a total of 206 are presented.

As it can be seen only one off-target epitope has been identified and ranked with the safety score below 3 (i.e. FLLMFIKQL, ranked with 2.55.)

In the final step there is presented mRNA expression in TPMs and protein expression level of the putative off-target epitopes in a figure format. Furthermore, a histogram with the distribution of all the safety scores is generated. Fig.8A, shows the exemplary outputs for said step.

VALIDATION OF THE METHOD

Said 4 clinically validated cases of TCRs targeting tumor associated antigens mentioned above have been used for validation of the method according to the invention. In 3 of these cases cross reactive antigens were identified empirically (for epitopes originating from MAGEA3 and AFP genes). In the last case the method according to the invention has been tested with a very promising target epitope shared in multiple types of cancer NY-ESO-1, for which as of now no off-target toxicity was observed..

The target epitopes and their respective cross reactive epitopes used for validation of the method according to the invention, together with their properties and status obtained in previous studies are presented in the below Table 5.

Table 5

| Case no. & status | Targeted peptide | Off-target peptide | HLA type | Clinical status | Toxic side effects on the patient |
|---|---|---|---|---|---|
| **1) toxic** | **KVAELVHFL** *MAGEA3* | **KMAELVHFL** *MACEA12* | AO2:O1 | terminated after phase 2 due to adverse events related to study treatment | mental status changes, comas, death |
| **2) toxic** | **EVDPIGHLY** *MAGEA3* | **ESDPIVAQY** *TTN* | AO1:O1 | terminated after phase 2 due to adverse events related to study treatment | myocardial damage leading to death |
| **3) unknown status** | **FMNKFIYEI** *AFP* | **ILNKFIPDI** *RCL1* | AO2:O1 | off-target based on X-scan, ongoing clinical trials | up to date no adverse effects were shown |
| **4) safe** | **SLLMWtTQV** *NY-ESO-1* | None | AO2:O1 | completed phase 2 | up to date no adverse effects were shown |

*Case 1*

Morgan et al. described a cell therapy based on TCR engineered T-cells against KVAELVHFL epitope derived from MAGEA3. The clinical study was performed on 9 HLA-A*0201 positive patients with metastatic cancer expressing MAGEA3 and MAGEA12 see Morgan RA, Chinnasamy N, Abate-Daga D, Gros A, Robbins PF, Zheng Z, Dudley ME, Feldman SA, Yang JC, Sherry RM, Phan GQ, Hughes MS, Kammula US, Miller AD, Hessman CJ, Stewart AA, Restifo NP, Quezado MM, Alimchandani M, Rosenberg AZ, Nath A, Wang T, Bielekova B, Wuest SC, Akula N, McMahon FJ, Wilde S, Mosetter B, Schendel DJ, Laurencot CM, Rosenberg SA. "Cancer regression and neurological toxicity following anti-MAGE-A3 TCR gene therapy". J Immunother. 2013 Feb;36(2):133-51.

Unfortunately, three patients developed considerable side effects. Two patients suffered from coma that ultimately resulted in their death. One patient developed Parkison-like symptoms that lasted for 4 weeks after the administration of the drug. Based on molecular assays the authors showed that genes from the same family as the target, specifically MAGEA12 and to a lower degree MAGEA1, MAGEA8, and MAGEA9 are expressed in the brain and are probably responsible for the off-target toxicity effect.

By the method according to the present invention, when applied to analyze the initial target, 300 putative off-target epitopes have been identified with high probability of being presented on the cell surface. 24 off-target epitopes had a safety score below < 3. Specifically, putative off-target epitopes originating from MAGEA12, MAGEA8, and MAGEA9 genes are labeled by the method according to the invention as off-target epitopes with high potential to generate off-target toxicity (highest possible score 0) (see Fig. 5A). Furthermore, in both MAGEA12 and MAGEA8 mRNA expression is observed in different parts of the brain (see Fig. 5B).

**Case** 2

Linette et al. described a case of off-target toxicity in a clinical study conducted on 4 patients with myeloma and melanoma, see Linette GP, Stadtmauer EA, Maus MV, Rapoport AP, Levine BL, Emery L, Litzky L, Bagg A, Carreno BM, Cimino PJ, Binder-Scholl GK, Smethurst DP, Gerry AB, Pumphrey NJ, Bennett AD, Brewer JE, Dukes J, Harper J, Tayton-Martin HK, Jakobsen BK, Hassan NJ, Kalos M, June CH. "Cardiovascular toxicity and titin cross-reactivity of affinity-enhanced T cells in myeloma and melanoma". Blood. 2013 Aug 8;122(6):863-71.

The engineered T cells against MAGEA3 epitope (EVDPIGHLY) were presented on HLA-A*01. The first 2 patients that received the drug developed cardiogenic shock and as a result died within the next few days. Interestingly, the off-target epitope was not identified prior to the clinical studies. Only, after performing the experiments on in vitro beating cardiomyocytes, a titin (TTN) epitope was identified to be responsible for the toxicity effects.

On the other hand, by performing the computer implemented method according to the invention, the Authors were able to identify 85 potential off-target epitopes among which nine had a safety score < 3 (see Fig.6A). Importantly, the epitope originating from TTN was one of the top hits with a safety score of 0. The expression for both the protein and mRNA for TTN was found in muscle and cardiac cells (see Fig. 6B).

**Case 3**

Cai et al. tested preclinically engineered T cells against AFP 158 TAA (FMNKFIYEI), see Cai L, Caraballo Galva LD, Peng Y, Luo X, Zhu W, Yao Y, Ji Y, He Y. "Preclinical Studies of the Off-Target Reactivity of AFP 158 -Specific TCR Engineered T Cells". Front Immunol. 2020 Apr 27;11 :607. Based on an in vitro X-scan experiment, two off-target epitopes that could activate the T-cell were identified: $ENPP1_{436}$ and $RCL1_{215}$. However, according to Cai et al. in humans $ENPP1_{436}$ is not processed nor presented on the human cells.

Results of the computer implemented method according to the invention pointed 39 putative off-target epitopes for this target. As much as 8 of them were characterized with a safety score < 3 (see Fig. 7A). Among them an experimentally identified epitope; $RCL1_{215}$ with a safety score of 2.47 indicating its high off-target toxicity potential (see Fig. 7B) was identified. RCL1 mRNA expression was found to be uniform across tissues.

*Case 4*

Stadmauer et al. performed a clinical study on 25 high-risk multiple myeloma patients using T cells engineered against NY-ESO-1 (SLLMWITQV), see Stadtmauer EA, Faitg TH, Lowther DE, Badros AZ, Chagin K, Dengel K, Iyengar M, Melchiori L, Navenot JM, Norry E, Trivedi T, Wang R, Binder GK, Amado R, Rapoport AP. "Long-term safety and activity of NY-ESO-1 SPEAR T cells after autologous stem cell transplant for myeloma". Blood Adv. 2019 Jul 9;3(13):2022-2034.

NY-ESO-1 is a cancer-testis antigen (CTAs) with expression in multiple types of cancer. NY-ESO-1 is considered as one of the most promising engineered T-cells for cancer immunotherapy as no adverse off-target toxicity was identified and the therapy increases survival of the patients considerably.

This particular epitope is presented on HLA-A*02:01. For that peptide-HLA combination, by performing the method according to the invention, the Authors found 206 putative off-target epitopes. However, out of all the off-target epitopes only one epitope derived from LRBA (FLLMFIKQL) had a safety score <3 (Fig. 8A).

**Case 5**

**off-target epitopes trends**

*TAA epitopes vs Virus epitopes*

The method according to the invention has been tested on 148 epitopes from TAA and viruses presented either on HLA-A*02:01 or HLA-A*01:01. As expected, due to the evolutionary distance between the tested peptides the number of putative off-target epitopes for TAA epitopes was higher (31854) than off-target epitopes peptides of viral origin (22279). The t-test conducted on the safety scores suggest a significant difference between the distributions (mean TAA=7.39688, mean Virus=7.622937, p-value < 2.2e-16). On the other hand, Cohen's d (-0.1514) suggest rather negligible difference. The above values, together with the similar shapes of distributions of TAA and Virus off-target epitopes, suggest that the distribution of safety scores is comparable between the two groups.

*TAA epitopes vs frameshift epitopes*

Lastly, the method according to the invention was tested on 16 frameshift epitopes and 16 TAA peptides, predicted by our model to be presented on HLA-A*02:01.

Importantly, only 336 putative off-target epitopes were identified for the frameshift derived fragments, while as much as 3911 cross-reactive peptides appeared in the TAA group. The t-test conducted on the safety scores was significant with a p-value = 2.544e-06 (mean TAA=7.219123, mean Frameshift=7.675771), while Cohen's d value was low (0.2816135).

Interestingly, no off-target epitopes derived from frameshift variants with a score below 3.5 were found.

**Claims**

1. A computer implemented method for predicting the occurrence of the off-target toxicity caused by similarity between a target epitope and off-target epitopes, the method comprising:

   a) providing said target epitope of a predefined length and its corresponding HLA type,
   b) identifying a set of all putative off-target epitopes present in a reference proteome having the same length as the predefined length of said target epitope,
   c) extracting a subset of off-target epitopes from the identified set of all putative off-target epitopes having surface HLA presentation probability for said HLA type above a presentation probability threshold value with the use of a presentation model,
   d) ranking said extracted subset of off-target epitopes for its potential for toxicity by calculating a numerical score value for each off-target epitope indicative of a degree of similarity between said target epitope and each said off-target epitope, based on at least one physico-chemical property of the amino acids at predefined fixed positions interacting with antigen-recognition receptors.

2. The method according to claim 1, wherein said at least one physico-chemical property relates to at least one among: amino acid secondary structure prediction beta-strand, aperiodic structure and alpha helix, sizes of radii and volume of atomic groups, relation of amino acid composition depending on protein localization and inter-residue protein contact energies, advantageously at least one among: isoelectric point, polarity, molecular weight, average accessible surface area, mutability, hydration potential, refractivity, optical activity, flexibility.

3. The method according to claim 1, wherein defining fixed positions in amino acid sequence of the target epitope and the off-target epitope responsible for interacting with antigen-recognition receptors comprises consulting a residues database with amino acid positions important for interaction with antigen-recognition receptors depending on the HLA type relating to said target epitope and off-target epitope, said database being generated based on at least results of A-scan and/or X-scan.

4. The method according to claim 4, wherein said database with amino acid positions important for interaction with antigen-recognition receptors depending on the HLA type relating to said target epitope and off-target epitope is additionally generated with the data relating to non-HLA faced amino acids.

5. The method according to any of the preceding claims, wherein the step d) of ranking off-target epitopes by obtaining a numerical score value for each said off-target epitope comprises:

- limiting amino acids sequences for said target epitope and for said each off-target epitope by deleting amino acids in their sequences at predefined fixed positions that are not interacting with antigen-recognition receptors,
- for said target epitope and for said each off-target epitope determining a vector of the size $n = p_n * r_n$, where $p_n$ is the number of predefined residues and $r_n$ is the number of physico-chemical properties used for scoring,
- then calculating the distance x between said target epitope and said off-target epitope according to the following norm:

$$x = \sqrt{\sum_{i=1}^{n} (e_i - p_i)^2}$$

where $e_i$ and $p_i$ represent the *i-th* amino acids physico-chemical property from compared target epitope e and off-target epitope *p,* and n is equal to the length of the vector $n = p_n * r_n$,
- receiving the numerical score value x for each said off-target epitope being said calculated distance, wherein said numerical score x ranges between 0 and 40, advantageously between 0 and 14, wherein if the value of the numerical score value for the off-target under assessment is close to zero it means that said off-target epitope and the target epitope are almost identical with high probability for toxicity to occur and wherein if the value of the safety score for the off-target under assessment is above a threshold value it means that the off-target under assessment has a low potential for toxicity.

6. The method according to any of preceding claims, wherein the predefined length of the target epitope is between 8 to 11 amino acids.

7. The method according to claim 1, wherein the step b) of identifying a set of all putative off-target epitopes present in a reference proteome having the same length as the predefined length of the target epitope comprises:

- generating an artificial set of amino acids sequences having at least 5 amino acids in common with said target epitope according to a formula:

$$\sum_{k=0,1...n} \frac{N!}{(N!-k!)k!} * 20^k.$$

wherein *N* is the predefined length of the target epitope and *k is* the number of admissible mismatches in amino acids sequence,
- checking occurrence of said artificially generated set of epitopes in the reference proteome,
- receiving a set of all naturally existing putative off-target epitopes by selecting only those artificially generated off-target epitopes which are present in said reference proteome.

8. The method according to claim 1, wherein before the step c) the method further comprises enlarging said set of all putative off-target epitopes through addition of epitopes derived from frequent genetic variants.

9. The method according to claim 8, wherein enlarging said set of all putative off-target epitopes through addition of epitopes derived from frequent genetic variants comprises for each off-target epitope within said of all putative off-target epitopes:

- determining an ENSG identificator based on its uniprot identifier,
- finding genome region, which codes said epitope using said ENSG identificator, along with the start and end position of the off-target epitope in the found genome region
- consulting the gnomAD database for mutations in said genome region
- transcription of each frequent mutation in said genome region into an additional naturally existing off-target epitope

- receiving an enlarged set of all naturally existing putative off-target epitopes by adding said additional naturally existing epitopes resulting from said mutations.

10. The method according to any of the preceding claims, wherein the step c) further comprises a preceding or subsequent substep of selecting only a subset of off-target epitopes having binding affinity level below a binding affinity threshold value, advantageously the binding affinity threshold value is equal to the IC50 value equal to 2000 nM.

11. The method according to claim 1, wherein in the step c) the presentation probability threshold value is equal to 50%.

12. The method according to claim 1, wherein in the step c) said presentation model is a trained presentation model and is based on at least one artificial neural network, and wherein the model is trained using datasets consisting of mass-spectroscopy experiments results conducted on monoallelic human cell line.

13. The method according to claim 1, wherein it further comprises the step of identifying mRNA and peptide tissue specific expression for each gene derived from a presented off-target epitope within said extracted subset along with indication of tissue sensitive to off-target toxicity caused by said each off-target epitope.

14. A computer-readable storage medium having instructions encoded thereon which when executed by a processor, cause the processor to perform the method according to claim 1 to 13.

15. A system having at least one processor and one memory configured to perform the method according to claim 1 to 13.

**INPUT**

- target epitope
- HLA type

Search of similiar sequences from the human reference proteome

Similarity scoring of off-target epitopes based on TCR faced amino acids

Addition of frequent SNP-derived epitopes

Similarity scoring of off-target epitopes based on TCR faced amino acids

Removal of off-target epitopes based on pHLA presentation and binding

Addition of mRNA and peptide tissue specific expression data for each off-target epitope

**OUTPUT**

Table with prioritized and scored cross-reactive antigens and their expression patterns

**Fig. 1**

**INPUT (output from previous step)**

- uniprot identificator
  e.g. sp|Q96P26|5NT1B_HUMAN
- epitope start position in the protein: 412
- epitope end position in the protein: 420
- epitope sequence: GKDPIGYLK

Identification of corresponding ENSG identificator
e.g. ENSG00000185013

Identification of genomic positions coding the epitope
in question e.g.
start: 2:18576837, end: 2:18576863

Verification if the DNA sequence, of the chosen region
e.g. 2:18576837 - 18576863, translates to the valid
epitope sequence, e.g. GKDPIGYLK

Query the GnomAD database for SNPs (single-
nucleotide polymorphism), in the genomic region in
question, with a frequency above the given threshold

Are there any SNPs present?

Yes

For every SNP:

Translation of the mutated DNA sequence into a novel
protein sequence that includes the frequent SNP

Extraction of the mutated peptide sequence from the
novel protein sequence

**OUTPUT**

Addition of the novel mutated epitopes with the
frequency of the mutations to the list of putative
epitopes

**Fig. 2**

**Trained presentation model**

Training data

Datasets consisting of mass-spectroscopy experiments results, conducted on monoallelic human cell lines, containing sequences of peptides being presented on cell surface

Algorithm

Neural network used to extract patterns from the training data, that allows for identification of presented peptides

Predictive model

$f(x)$

A function that uses recognized patterns to predict presentation probability for previously unseen peptide sequences

List of putative off-target epitopes

List of putative off-target epitopes with predicted probability of being presented above given threshold

**Fig. 3**

EP 4 379 726 A1

**INPUT (output from previous step)**

- target epitope sequence
  e.g. KLNKIISEI
- off-target epitope sequence
  e.g. FMNKFIYEI
- HLA-type
  e.g. HLA-B*07:02

Selection of TCR-faced amino acids in target and off-target epitopes for a defined HLA type
e.g. target epitope: KKIISE
e.g. off-target epitope: FKFIYE

Computation of the difference between the vectors representing target epitope and off-target epitope resulting in a single vector representing the similarity between target and off-target epitopes

Generation of vectors, consisting of the physico-chemical parameters of each TCR-face amino acid, for the target epitope and the off-target epitope.

Computation of the Frobenius norm on the vector representing the similarity between the target and off-target epitopes resulting in a numerical score value.

**OUTPUT**

A numerical score value for off-target epitope in question, that describes the degree of similarity towards the target epitope, which allows for the estimation of the risk of the off-target toxicity.

**Fig. 4**

Fig. 5a

Fig.5b

**Fig. 6a**

**Fig.6b**

**Fig. 7a**

**Fig.7b**

**Fig.8a**

**ardigen**

ANALYSIS START

Target ⓘ

EVDPIGHLY ✓

HLA ⓘ

A01:01 ✓

Analyse

Clear

OFF-TARGET ESTIMATION

1. Cross-Reactive Antigens
   1.1. Preliminary cross-reactivity results
2. mRNA & peptide expression level information
   2.1. Results table
   2.2. Safety score histogram
   2.3. Expression plot

## 2. mRNA & peptide expression level information

### 2.1. Results table

TISSUE TYPE

Choose an option ▾

MAXIMUM DISTANCE   10
0          10

MAXIMUM SCORE   13
0          13

| | GENEID ⇕ | GENE ⇕ | POS ⇕ | EPITOPE ⇕ | NEOPITOPE ⇕ | PRESENTATION_PROB ⇕ | SAFETY SCORE ⇕ |
|---|---|---|---|---|---|---|---|
| 1 | ENSG00000155657 | TTN | 2:178568174-178568200 | ESDPIVAQY | EVDPIGHLY | 0.8842 | 0 |
| 2 | ENSG00000221867 | MAGEA3 | X:152701334-152701360 | EVDPIGHLY | EVDPIGHLY | 0.9221 | 0 |
| 3 | ENSG00000197172 | MAGEA6 | X:152767123-152767149 | EVDPIGHVY | EVDPIGHLY | 0.8617 | 0 |
| 4 | ENSG00000176774 | MAGEB18 | X:26139481-26139507 | EVDPIRHYY | EVDPIGHLY | 0.9124 | 0 |
| 5 | ENSG00000170017 | ALCAM | 3:105534686-105534712 | EMDPVTQLY | EVDPIGHLY | 0.8950 | 1.1124 |
| 6 | ENSG00000139117 | CPNE8 | 12:38873037-38873050 | KSDPICVLY | EVDPIGHLY | 0.9890 | 1.5058 |
| 7 | ENSG00000154783 | FGD5 | 3:14932649-14932675 | EVGPIFHLY | EVDPIGHLY | 0.5999 | 2.4615 |
| 8 | ENSG00000173597 | SULT1B1 | 4:69733431-69733457 | EEHPILFLY | EVDPIGHLY | 0.5163 | 2.7006 |
| 9 | ENSG00000089472 | HEPH | X:66255124-66255141 | AVDPIKDMY | EVDPIGHLY | 0.9232 | 2.9051 |
| 10 | ENSG00000119946 | CNNM1 | 10:99360975-99360975 | EVEPFKSLY | EVDPIGHLY | 0.7709 | 3.0862 |
| 11 | ENSG00000177992 | SPATA31E1 | 9:87885382-87885408 | SSDPIWDLY | EVDPIGHLY | 0.9202 | 3.3173 |

Download

**Fig. 9**

Fig. 10

**Table 1b**

| gene_id | gene | pos | chr | gene_start | gene_end | epitope | rs_id | AF | reference _genome |
|---|---|---|---|---|---|---|---|---|---|
| ENSG00000188157 | AGRN | 1:1050431-1050457 | 1 | 1050431 | 1050457 | KMALEIVFL | rs17160775 | 0.9891745 | FALSE |
| ENSG00000132688 | NES | 1:156676854-156676880 | 1 | 156676854 | 156676880 | QAAELEREL | rs4278369 | 1 | FALSE |
| ENSG00000076321 | KLHL20 | 1:173766213-173766239 | 1 | 173766213 | 173766239 | AVAVLGGFF | rs7536773 | 1 | FALSE |
| ENSG00000185888 | PRSS38 | 1:227815801-227815827 | 1 | 227815801 | 227815827 | RVTALVHRQ | rs41315595 | 1 | FALSE |
| ENSG00000182077 | PTCHD3 | 10:27411915-27411929 | 10 | 27411915 | 27411929 | KKIEGVHFT | rs200200060 | 1 | FALSE |
| ENSG00000188385 | JAKMIP3 | 10:132149988-132150014 | 10 | 132149988 | 132150014 | KVVELLSEE | rs117391524 | 1 | FALSE |
| ENSG00000171813 | PWWP2B | 10:132405545-132405571 | 10 | 132405545 | 132405571 | YRAELVGEL | rs76595411 | 1 | FALSE |
| ENSG00000182103 | FAM181B | 11:82732935-82732961 | 11 | 82732935 | 82732961 | KGAEAVEFF | rs76427896 | 1 | FALSE |
| ENSG00000255374 | TAS2R43 | 12:11091663-11091689 | 12 | 11091663 | 11091689 | MVANLVPFT | rs76975331 | 1 | FALSE |
| ENSG00000187109 | NAP1L1 | 12:76060180-76060206 | 12 | 76060180 | 76060206 | KFYEEVHDL | rs79950675 | 1 | FALSE |
| ENSG00000125247 | TMTC4 | 13:100635093-100635119 | 13 | 100635093 | 100635119 | VVAERVLYL | rs946838 | 1 | FALSE |
| ENSG00000129566 | TEP1 | 14:20383853-20383879 | 14 | 20383853 | 20383879 | KVAPLVFFH | rs1760904 | 1 | FALSE |
| ENSG00000156414 | TDRD9 | 14:104014725-104014748 | 14 | 104014725 | 104014748 | EVAELYEQL | rs11160779 | 1 | FALSE |
| ENSG00000104044 | OCA2 | 15:27957614-27957640 | 15 | 27957614 | 27957640 | KVLALEHLL | rs151225947 | 0.0120631 | FALSE |
| ENSG00000069966 | GNB5 | 15:52154045-52154071 | 15 | 52154045 | 52154071 | QVAERVEAL | rs35581121 | 0.0271096 | FALSE |
| ENSG00000136383 | ALPK3 | 15:84816850-84816876 | 15 | 84816850 | 84816876 | EVAWLVYVL | rs11857356 | 0.811497 | FALSE |
| ENSG00000086504 | MRPL28 | 16:367732-367758 | 16 | 367732 | 367758 | CVAELIQQL | rs13226 | 1 | FALSE |
| ENSG00000167723 | TRPV3 | 17:3543523-3543549 | 17 | 3543523 | 3543549 | CMEELVELL | rs8066242 | 1 | FALSE |
| ENSG00000134760 | DSG1 | 18:31318322-31318348 | 18 | 31318322 | 31318348 | VVAVLFIFL | rs1426310 | 1 | FALSE |
| ENSG00000197563 | PIGN | 18:62102797-62102823 | 18 | 62102797 | 62102823 | KEELLVHLL | rs12326381 | 1 | FALSE |

**Fig. 11**

**Table 2b**

| gene_id | gene | pos | chr | gene_start | gene_end | epitope | rs_id | AF | reference_genome |
|---|---|---|---|---|---|---|---|---|---|
| ENSG00000187862 | TTC24 | 1:156582055-156582070 | 1 | 156582055 | 156582070 | GRRLLGHLY | rs6682716 | 1 | FALSE |
| ENSG00000119283 | TRIM67 | 1:231200232-231200258 | 1 | 231200232 | 231200258 | ENDPSGFLQ | rs139376072 | 1 | FALSE |
| ENSG00000242180 | OR51B5 | 11:5343039-5343065 | 11 | 5343039 | 5343065 | VVPPIRLLY | rs4910551 | 1 | FALSE |
| ENSG00000242180 | OR51B5 | 11:5343039-5343065 | 11 | 5343039 | 5343065 | IVPPIRPLY | rs12273630 | 1 | FALSE |
| ENSG00000261739 | GOLGA8S | 15:23363678-23363700 | 15 | 23363678 | 23363700 | EADGGGHLD | rs2344785 | 0.0100264 | FALSE |
| ENSG00000261739 | GOLGA8S | 15:23363678-23363700 | 15 | 23363678 | 23363700 | EGDGRGHLD | rs2880956 | 0.213101 | FALSE |
| ENSG00000183208 | GDPGP1 | 15:90241617-90241643 | 15 | 90241617 | 90241643 | PLDPEGHLH | rs75296384 | 0.0105314 | FALSE |
| ENSG00000118162 | KPTN | 19:47480964-47480990 | 19 | 47480964 | 47480990 | GNDPAIHLY | rs61743291 | 0.508148 | FALSE |
| ENSG00000218891 | ZNF579 | 19:55577975-55578001 | 19 | 55577975 | 55578001 | EVDSKAHLR | rs2292812 | 1 | FALSE |
| ENSG00000118960 | HS1BP3 | 2:20624790-20624816 | 2 | 20624790 | 20624816 | EVDPDEGLF | rs2305457 | 1 | FALSE |
| ENSG00000144031 | ANKRD53 | 2:70984980-70985006 | 2 | 70984980 | 70985006 | EVRPDGLGG | rs3796100 | 1 | FALSE |
| ENSG00000144031 | ANKRD53 | 2:70984980-70985006 | 2 | 70984980 | 70985006 | EVRPDGHGG | rs61732224 | 1 | FALSE |
| ENSG00000178026 | LRRC75B | 22:24589860-24589886 | 22 | 24589860 | 24589886 | PVDPISHDL | rs61737674 | 0.0136296 | FALSE |
| ENSG00000008226 | DLEC1 | 3:38116842-38116868 | 3 | 38116842 | 38116868 | EGVPSGHLY | rs75614900 | 1 | FALSE |
| ENSG00000010327 | STAB1 | 3:52516984-52517009 | 3 | 52516984 | 52517009 | EVDLCAHGH | rs79979130 | 1 | FALSE |
| ENSG00000146090 | RASGEF1C | 5:180136411-180136437 | 5 | 180136411 | 180136437 | EESTIGHLK | rs11546322 | 1 | FALSE |
| ENSG00000024048 | UBR2 | 6:42603588-42603608 | 6 | 42603588 | 42603608 | EEDPLVHLS | rs16895863 | 1 | FALSE |
| ENSG00000112214 | FHL5 | 6:96610695-96610721 | 6 | 96610695 | 96610721 | CMDCYNHLY | rs2252816 | 1 | FALSE |
| ENSG00000148290 | SURF1 | 9:133352573-133352599 | 9 | 133352573 | 133352599 | EVHLIGMVR | rs72619327 | 1 | FALSE |
| ENSG00000147381 | MAGEA4 | X:151924169-151924195 | X | 151924169 | 151924195 | EVDPTSNTY | rs1047251 | 1 | FALSE |

**Fig. 12**

Table 3b

| gene_id | gene | pos | chr | gene_start | gene_end | epitope | rs_id | AF | reference_genome |
|---|---|---|---|---|---|---|---|---|---|
| ENSG00000165406 | MARCH8 | 10:45458311-45458337 | 10 | 45458311 | 45458337 | FENKHGHGI | rs7908745 | 1 | FALSE |
| ENSG00000073910 | FRY | 13:32201996-32202022 | 13 | 32201996 | 32202022 | DTNREIYEI | rs9567420 | 1 | FALSE |
| ENSG00000187790 | FANCM | 14:45196457-45196483 | 14 | 45196457 | 45196483 | SKNKFIEQI | rs45557033 | 0.0213866 | FALSE |
| ENSG00000159248 | GJD2 | 15:34752553-34752579 | 15 | 34752553 | 34752579 | AKRKSIYEI | rs34964522 | 0.0830721 | FALSE |
| ENSG00000262628 | OR1D5 | 17:3062735-3062761 | 17 | 3062735 | 3062761 | MMNPFIYSL | rs2676564 | 0.621944 | FALSE |
| ENSG00000094661 | OR1I1 | 19:15087915-15087941 | 19 | 15087915 | 15087941 | MLNPFIYST | rs16980312 | 1 | FALSE |
| ENSG00000100316 | RPL3 | 22:39314197-39314208 | 22 | 39314197 | 39314208 | EINKKIYKI | rs11547996 | 0.0101482 | FALSE |
| ENSG00000185313 | SCN10A | 3:38698351-38698377 | 3 | 38698351 | 38698377 | FLVMFIYSI | rs6599242 | 1 | FALSE |
| ENSG00000221910 | OR2F2 | 7:143935737-143935763 | 7 | 143935737 | 143935763 | CANKFIDHI | rs13229174 | 1 | FALSE |

Fig. 13

**Table 4b**

| gene_id | gene | pos | chr | gene_start | gene_end | epitope | rs_id | AF | reference_genome |
|---|---|---|---|---|---|---|---|---|---|
| ENSG00000185842 | DNAH14 | 1:225080391-225080417 | 1 | 225080391 | 225080417 | LLLCDGTQV | rs115366080 | 1 | FALSE |
| ENSG00000138336 | TET1 | 10:68572807-68572833 | 10 | 68572807 | 68572833 | SVPMQGTQV | rs10823229 | 1 | FALSE |
| ENSG00000119919 | NKX2-3 | 10:99535176-99535202 | 10 | 99535176 | 99535202 | SLKLTSTQV | rs113459553 | 1 | FALSE |
| ENSG00000188620 | HMX3 | 10:123137444-123137470 | 10 | 123137444 | 123137470 | SLHLTETQV | rs60720577 | 1 | FALSE |
| ENSG00000205409 | OR52E6 | 11:5841412-5841438 | 11 | 5841412 | 5841438 | SLYMVVPLV | rs4357719 | 1 | FALSE |
| ENSG00000167552 | TUBA1A | 12:49185889-49185915 | 12 | 49185889 | 49185915 | SLLMERLSV | rs697624 | 1 | FALSE |
| ENSG00000102683 | SGCG | 13:23250636-23250662 | 13 | 23250636 | 23250662 | SLLLQSTQN | rs1800351 | 0.582518 | FALSE |
| ENSG00000103197 | TSC2 | 16:2064404-2064427 | 16 | 2064404 | 2064427 | SLLDIIEKV | rs34012042 | 1 | FALSE |
| ENSG00000160505 | NLRP4 | 19:55858213-55858239 | 19 | 55858213 | 55858239 | SLLIAIKPV | rs421810 | 1 | FALSE |
| ENSG00000115306 | SPTBN1 | 2:54622360-54622386 | 2 | 54622360 | 54622386 | DLLEWIEQT | rs2228365 | 0.595762 | FALSE |
| ENSG00000162994 | CLHC1 | 2:55177744-55177770 | 2 | 55177744 | 55177770 | QLLMSCPQV | rs13032294 | 1 | FALSE |
| ENSG00000168883 | USP39 | 2:85625539-85625544 | 2 | 85625539 | 85625544 | SSLEDITYV | rs2304564 | 1 | FALSE |
| ENSG00000153165 | RGPD3 | 2:106432950-106432976 | 2 | 106432950 | 106432976 | SLRKMIRQE | rs756675537 | 1 | FALSE |
| ENSG00000152086 | TUBA3E | 2:130194365-130194391 | 2 | 130194365 | 130194391 | SLLMEWLSV | rs62165074 | 1 | FALSE |
| ENSG00000155657 | TTN | 2:178795171-178795197 | 2 | 178795171 | 178795197 | PLLMCKTQA | rs16866538 | 1 | FALSE |
| ENSG00000124116 | WFDC3 | 20:45789901-45789927 | 20 | 45789901 | 45789927 | SLESWITAG | rs3746493 | 1 | FALSE |
| ENSG00000150995 | ITPR1 | 3:4814473-4814499 | 3 | 4814473 | 4814499 | TLLMCIVTV | rs711631 | 1 | FALSE |
| ENSG00000196578 | OR5AC2 | 3:98087455-98087481 | 3 | 98087455 | 98087481 | SLPECITQF | rs76749982 | 1 | FALSE |
| ENSG00000157426 | AASDH | 4:56371514-56371540 | 4 | 56371514 | 56371540 | SLLIVPTSV | rs6554354 | 1 | FALSE |
| ENSG00000138760 | SCARB2 | 4:76213481-76213507 | 4 | 76213481 | 76213507 | SLDCWITDK | rs72857097 | 1 | FALSE |

**Fig. 14**

EP 4 379 726 A1

**Table 1c**

| Epitope | Neoepitope | HLA_type | pres_prob | Safety_score | GENEID | gene | pos | rs_id | AF | reference_genome |
|---|---|---|---|---|---|---|---|---|---|---|
| KVAPLEAKL | KVAELVHFL | A02:01 | 0,53321326 | 9,211816713 | ENSG00000165182 | CXorf58 | X:23915730-23915756 | 0 | 1 | TRUE |
| KVVSLVHFL | KVAELVHFL | A02:01 | 0,5848929 | 4,655895885 | ENSG00000176774 | MAGEB18 | X:26139313-26139339 | 0 | 1 | TRUE |
| SVADLAHVL | KVAELVHFL | A02:01 | 0,79953474 | 4,809028711 | ENSG00000078589 | P2RY10 | X:78960749-78960775 | 0 | 1 | TRUE |
| KLVELEHTL | KVAELVHFL | A02:01 | 0,9434157 | 7,196766316 | ENSG00000204019 | CT83 | X:116461789-116461815 | 0 | 1 | TRUE |
| MVDELVLLL | KVAELVHFL | A02:01 | 0,6981364 | 4,18538062 | ENSG00000182195 | LDOC1 | X:141176734-141176760 | 0 | 1 | TRUE |
| KVAELVQFL | KVAELVHFL | A02:01 | 0,84716237 | 0 | ENSG00000165509 | MAGEC3 | X:141881446-141881472 | 0 | 1 | TRUE |
| KVAELVEFL | KVAELVHFL | A02:01 | 0,86897284 | 0 | ENSG00000046774 | MAGEC2 | X:142203532-142203558 | 0 | 1 | TRUE |
| KVAELVHFL | KVAELVHFL | A02:01 | 0,9533381 | 0 | ENSG00000267978 | MAGEA9B | X:149582823-149582849 | 0 | 1 | TRUE |
| KIIDLVHLL | KVAELVHFL | A02:01 | 0,9379009 | 5,307172225 | ENSG00000185247 | MAGEA11 | X:149716159-149716185 | 0 | 1 | TRUE |
| KVAELVHFL | KVAELVHFL | A02:01 | 0,9533381 | 0 | ENSG00000123584 | MAGEA9 | X:149786541-149786567 | 0 | 1 | TRUE |
| KVAELVRFL | KVAELVHFL | A02:01 | 0,5749293 | 0 | ENSG00000156009 | MAGEA8 | X:149884615-149884641 | 0 | 1 | TRUE |
| KVDELAHFL | KVAELVHFL | A02:01 | 0,6771199 | 4,564736975 | ENSG00000147381 | MAGEA4 | X:151924001-151924027 | 0 | 1 | TRUE |
| KVTDLVQFL | KVAELVHFL | A02:01 | 0,8131012 | 3,70162729 | ENSG00000124260 | MAGEA10 | X:152135186-152135212 | 0 | 1 | TRUE |
| KVAELVHFL | KVAELVHFL | A02:01 | 0,9533381 | 0 | ENSG00000221867 | MAGEA3 | X:152701166-152701192 | 0 | 1 | TRUE |
| KMVELVHFL | KVAELVHFL | A02:01 | 0,96330297 | 3,266894043 | ENSG00000183305 | MAGEA2B | X:152717440-152717466 | 0 | 1 | TRUE |
| KMAELVHFL | KVAELVHFL | A02:01 | 0,98829496 | 0 | ENSG00000213401 | MAGEA12 | X:152736495-152736521 | 0 | 1 | TRUE |
| KMVELVHFL | KVAELVHFL | A02:01 | 0,96330297 | 3,266894043 | ENSG00000268606 | MAGEA2 | X:152751004-152751030 | 0 | 1 | TRUE |
| KLAELARQL | KVAELVHFL | A02:01 | 0,7529267 | 5,203305104 | ENSG00000213397 | HAUS7 | X:153456304-153456330 | 0 | 1 | TRUE |
| AIAGLVVFL | KVAELVHFL | A02:01 | 0,79409194 | 4,36383443 | ENSG00000101986 | ABCD1 | X:153725999-153726025 | 0 | 1 | TRUE |
| RVADLVHIL | KVAELVHFL | A02:01 | 0,8749264 | 3,086244638 | ENSG00000184216 | IRAK1 | X:154019474-154019500 | 0 | 1 | TRUE |

**Fig. 15**

**Table 2c**

| Epitope | Neoepitope | HLA_type | pres_prob | Safety_score | GENEID | gene | pos | rs_id | AF | reference _genome |
|---|---|---|---|---|---|---|---|---|---|---|
| VLDFITHLY | EVDPIGHLY | A01:01 | 0,9675326 | 8,321509762 | ENSG00000068120 | COASY | 17:42562812-42562838 | 0 | 1 | TRUE |
| ELDPTFGLY | EVDPIGHLY | A01:01 | 0,8863179 | 3,662747019 | ENSG00000161914 | ZNF653 | 19:11496050-11496076 | 0 | 1 | TRUE |
| KVDGFTHLY | EVDPIGHLY | A01:01 | 0,97909874 | 4,606010425 | ENSG00000269058 | CALR3 | 19:16484092-16484115 | 0 | 1 | TRUE |
| GNDPAIHLY | EVDPIGHLY | A01:01 | 0,8535816 | 5,808605516 | ENSG00000118162 | KPTN | 19:47480964-47480990 | 0 | 1 | TRUE |
| GNDPAIHLY | EVDPIGHLY | A01:01 | 0,8535816 | 5,808605516 | ENSG00000118162 | KPTN | 19:47480964-47480990 | rs61743291 | 0,508148 | FALSE |
| VVDPSSNLY | EVDPIGHLY | A01:01 | 0,9417377 | 6,906374936 | ENSG00000144191 | CNGA3 | 2:98389686-98389712 | 0 | 1 | TRUE |
| ERDKIFLLY | EVDPIGHLY | A01:01 | 0,7847913 | 5,350944724 | ENSG00000135951 | TSGA10 | 2:99108839-99108865 | 0 | 1 | TRUE |
| RVDQNGVLY | EVDPIGHLY | A01:01 | 0,9369509 | 7,658329616 | ENSG00000204406 | MBD5 | 2:148458840-148458866 | 0 | 1 | TRUE |
| EVDEITLWY | EVDPIGHLY | A01:01 | 0,88899124 | 5,595237318 | ENSG00000078098 | FAP | 2:162189162-162189172 | 0 | 1 | TRUE |
| ESDPIVAQY | EVDPIGHLY | A01:01 | 0,88424087 | 0 | ENSG00000155657 | TTN | 2:178568174-178568200 | 0 | 1 | TRUE |
| WVDPIDSTY | EVDPIGHLY | A01:01 | 0,88710237 | 5,669236978 | ENSG00000151689 | INPP1 | 2:190366880-190366895 | 0 | 1 | TRUE |
| SVDKIEVLY | EVDPIGHLY | A01:01 | 0,9624819 | 6,295822365 | ENSG00000173692 | PSMD1 | 2:231062308-231062321 | 0 | 1 | TRUE |
| KVDGIYLLY | EVDPIGHLY | A01:01 | 0,993906 | 4,219771559 | ENSG00000157551 | KCNJ15 | 21:38299385-38299411 | 0 | 1 | TRUE |
| EVDPDTILK | EVDPIGHLY | A01:01 | 0,7347156 | 5,292384925 | ENSG00000100227 | POLDIP3 | 22:42585857-42585883 | 0 | 1 | TRUE |
| EVGPIFHLY | EVDPIGHLY | A01:01 | 0,5998936 | 2,461471654 | ENSG00000154783 | FGD5 | 3:14932649-14932675 | 0 | 1 | TRUE |
| TVDENGRLY | EVDPIGHLY | A01:01 | 0,7030097 | 8,206852243 | ENSG00000244607 | CCDC13 | 3:42757142-42757168 | 0 | 1 | TRUE |
| VVDNLQHLY | EVDPIGHLY | A01:01 | 0,9743714 | 7,006365924 | ENSG00000010322 | NISCH | 3:52471837-52471863 | 0 | 1 | TRUE |
| EMDPVTQLY | EVDPIGHLY | A01:01 | 0,8950302 | 1,11243857 | ENSG00000170017 | ALCAM | 3:105534686-105534712 | 0 | 1 | TRUE |
| EVDVPIKLY | EVDPIGHLY | A01:01 | 0,7705895 | 9,55693102 | ENSG00000154928 | EPHB1 | 3:134951938-134951964 | 0 | 1 | TRUE |
| FVDPGERLY | EVDPIGHLY | A01:01 | 0,9514075 | 7,309697298 | ENSG00000158092 | NCK1 | 3:136945645-136945671 | 0 | 1 | TRUE |

**Fig. 16**

EP 4 379 726 A1

**Table 3c**

| Epitope | Neoepitope | HLA_type | pres_prob | Safety_score | GENEID | gene | pos | rs_id | AF | reference_genome |
|---|---|---|---|---|---|---|---|---|---|---|
| MMNPFIYRL | FMNKFIYEI | A02:01 | 0,939238 | 5,889495537 | ENSG00000262628 | OR1D5 | 17:3062735-3062761 | 0 | 0,378056 | TRUE |
| MMNPFIYSL | FMNKFIYEI | A02:01 | 0,9040544 | 6,295822365 | ENSG00000262628 | OR1D5 | 17:3062735-3062761 | rs2676564 | 0,621944 | FALSE |
| MMNPFIYSL | FMNKFIYEI | A02:01 | 0,9040544 | 6,295822365 | ENSG00000184166 | OR1D2 | 17:3092124-3092150 | 0 | 1 | TRUE |
| MMNPFIYSL | FMNKFIYEI | A02:01 | 0,9040544 | 6,295822365 | ENSG00000161807 | OR7G1 | 19:9114888-9114914 | 0 | 1 | TRUE |
| MLNPFIYSI | FMNKFIYEI | A02:01 | 0,9385321 | 6,295822365 | ENSG00000094661 | OR1I1 | 19:15087915-15087941 | 0 | 1 | TRUE |
| FLQKDIFEI | FMNKFIYEI | A02:01 | 0,923584 | 5,33813357 | ENSG00000196357 | ZNF565 | 19:36183684-36183710 | 0 | 1 | TRUE |
| FLKKEIYEI | FMNKFIYEI | A02:01 | 0,8691666 | 5,838571096 | ENSG00000186017 | ZNF566 | 19:36449943-36449969 | 0 | 1 | TRUE |
| TMNKHLYTI | FMNKFIYEI | A02:01 | 0,7204921 | 5,191056596 | ENSG00000183091 | NEB | 2:151655360-151655374 | 0 | 1 | TRUE |
| RLIKFIYNI | FMNKFIYEI | A02:01 | 0,9434101 | 6,08484083 | ENSG00000198925 | ATG9A | 2:219225125-219225151 | 0 | 1 | TRUE |
| FLKKYLYEI | FMNKFIYEI | A02:01 | 0,717032 | 4,492507553 | ENSG00000163631 | ALB | 4:73408795-73408805 | 0 | 1 | TRUE |
| FMNKFIYEI | FMNKFIYEI | A02:01 | 0,88626754 | 0 | ENSG00000081051 | AFP | 4:73440803-73440813 | 0 | 1 | TRUE |
| RMNGFIDQI | FMNKFIYEI | A02:01 | 0,6704619 | 4,606431588 | ENSG00000138663 | COPS4 | 4:83068474-83068500 | 0 | 1 | TRUE |
| SMQQFLYEI | FMNKFIYEI | A02:01 | 0,54375994 | 3,549045959 | ENSG00000197283 | SYNGAP1 | 6:33441234-33441260 | 0 | 1 | TRUE |
| FQVKKIYEL | FMNKFIYEI | A02:01 | 0,87115073 | 6,963398154 | ENSG00000171467 | ZNF318 | 6:43338130-43338156 | 0 | 1 | TRUE |
| TLNKFIEEL | FMNKFIYEI | A02:01 | 0,9189298 | 0 | ENSG00000112245 | PTP4A1 | 6:63576965-63576985 | 0 | 1 | TRUE |
| KLNKIISEI | FMNKFIYEI | A02:01 | 0,8242291 | 1,84630984 | ENSG00000112333 | NR2E1 | 6:108180836-108180862 | 0 | 1 | TRUE |
| RMNGIIYEI | FMNKFIYEI | A02:01 | 0,87226796 | 4,487221428 | ENSG00000104237 | RP1 | 8:54628437-54628463 | 0 | 1 | TRUE |
| ILNKFIPDI | FMNKFIYEI | A02:01 | 0,6569327 | 2,47306408 | ENSG00000120158 | RCL1 | 9:4841290-4841316 | 0 | 1 | TRUE |
| FMAVFILEV | FMNKFIYEI | A02:01 | 0,789452 | 6,369782442 | ENSG00000164946 | FREM1 | 9:14851515-14851541 | 0 | 1 | TRUE |
| KINQFIEEI | FMNKFIYEI | A02:01 | 0,6471091 | 1,674189247 | ENSG00000096872 | IFT74 | 9:27029044-27029070 | 0 | 1 | TRUE |

**Fig. 17**

Table 4c

| Epitope | Neoepitope | HLA_type | pres_prob | Safety_score | GENEID | gene | pos | rs_id | AF | reference_genome |
|---|---|---|---|---|---|---|---|---|---|---|
| SLLQDITMI | SLLMWITQV | A02:01 | 0,7948284 | 6,773147214 | ENSG00000187695 | RP11-723O4.6 | 3:128945280-128945306 | 0 | 0,170778 | TRUE |
| QLSTVITQV | SLLMWITQV | A02:01 | 0,97991765 | 7,161119098 | ENSG00000175054 | ATR | 3:142503382-142503408 | 0 | 1 | TRUE |
| NLLPSITDV | SLLMWITQV | A02:01 | 0,58849263 | 8,681191046 | ENSG00000197386 | HTT | 4:3140598-3140624 | 0 | 1 | TRUE |
| SLLIVPTSV | SLLMWITQV | A02:01 | 0,86181664 | 9,02642844 | ENSG00000157426 | AASDH | 4:56371514-56371540 | 0 | 1 | TRUE |
| SLLIVPTSV | SLLMWITQV | A02:01 | 0,86181664 | 9,02642844 | ENSG00000157426 | AASDH | 4:56371514-56371540 | rs6554354 | 1 | FALSE |
| SLLLLVTSV | SLLMWITQV | A02:01 | 0,75656533 | 6,915468387 | ENSG00000138760 | SCARB2 | 4:76175883-76175909 | 0 | 1 | TRUE |
| SLDALITHV | SLLMWITQV | A02:01 | 0,9569877 | 8,48639691 | ENSG00000187758 | ADH1A | 4:99279479-99279505 | 0 | 1 | TRUE |
| SLDALITHV | SLLMWITQV | A02:01 | 0,9569877 | 8,48639691 | ENSG00000196616 | ADH1B | 4:99310818-99310844 | 0 | 1 | TRUE |
| WLLNDIFQV | SLLMWITQV | A02:01 | 0,9452926 | 6,711608295 | ENSG00000205301 | MGAT4D | 4:140456616-140456642 | 0 | 0,9894548 | TRUE |
| FLLMFIKQL | SLLMWITQV | A02:01 | 0,5654521 | 2,551012227 | ENSG00000198589 | LRBA | 4:150897748-150897774 | 0 | 1 | TRUE |
| SLLQSILQL | SLLMWITQV | A02:01 | 0,92823565 | 6,175730739 | ENSG00000052795 | FNIP2 | 4:158891593-158891619 | 0 | 1 | TRUE |
| SLPFLIYQV | SLLMWITQV | A02:01 | 0,77273756 | 9,075529519 | ENSG00000164128 | NPY1R | 4:163326021-163326047 | 0 | 1 | TRUE |
| SGLLNITKV | SLLMWITQV | A02:01 | 0,55971736 | 6,389173601 | ENSG00000198948 | MFAP3L | 4:169992263-169992289 | 0 | 1 | TRUE |
| SLLGWILSI | SLLMWITQV | A02:01 | 0,8241344 | 5,934602524 | ENSG00000185758 | CLDN24 | 4:183322352-183322378 | 0 | 1 | TRUE |
| SLLKHMLQV | SLLMWITQV | A02:01 | 0,95150757 | 6,089091187 | ENSG00000132356 | PRKAA1 | 5:40767501-40767527 | 0 | 1 | TRUE |
| SLRHIIAQV | SLLMWITQV | A02:01 | 0,90785015 | 6,338456841 | ENSG00000145721 | LIX1 | 5:97142532-97142558 | 0 | 1 | TRUE |
| SLLDSSNQV | SLLMWITQV | A02:01 | 0,5488955 | 8,233316494 | ENSG00000145723 | GIN1 | 5:103087916-103087942 | 0 | 1 | TRUE |
| SLFKYITVV | SLLMWITQV | A02:01 | 0,9903762 | 6,917645834 | ENSG00000145725 | PPIP5K2 | 5:103133540-103133566 | 0 | 1 | TRUE |
| SVLGAITSV | SLLMWITQV | A02:01 | 0,9294084 | 8,097082146 | ENSG00000120705 | ETF1 | 5:138518720-138518746 | 0 | 1 | TRUE |
| SLLELVEQV | SLLMWITQV | A02:01 | 0,99134415 | 6,595183892 | ENSG00000113073 | SLC4A9 | 5:140360954-140360972 | 0 | 1 | TRUE |

**Fig. 18**

| | Europäisches<br>Patentamt<br>European<br>Patent Office<br>Office européen<br>des brevets | **EUROPEAN SEARCH REPORT** | Application Number<br>**EP 22 46 1636** |

## DOCUMENTS CONSIDERED TO BE RELEVANT

| Category | Citation of document with indication, where appropriate,<br>of relevant passages | Relevant<br>to claim | CLASSIFICATION OF THE<br>APPLICATION (IPC) |
|---|---|---|---|
| X | DATABASE EMBASE [Online]<br>ELSEVIER SCIENCE PUBLISHERS, AMSTERDAM,<br>NL;<br>1 May 2021 (2021-05-01),<br>MURCIA PIENKOWSKI V A ET AL: "OFF-TARGET<br>TOXICITY PREDICTION IN CELLULAR CANCER<br>IMMUNOTHERAPIES",<br>XP002809285,<br>Database accession no. EMB-002012003154 | 1-7,<br>10-12,<br>14,15 | INV.<br>G16B35/20<br>G16B40/20 |
| Y<br>A | * the whole document *<br><br>& MURCIA PIENKOWSKI V A ET AL:<br>"OFF-TARGET TOXICITY PREDICTION IN<br>CELLULAR CANCER IMMUNOTHERAPIES",<br>CYTOTHERAPY - ABSTRACTS OF THE 27TH ANNUAL<br>ISCT MEETING MAY 25-28, 2021 20210501<br>ELSEVIER B.V. NLD,<br>vol. 23, no. 5, Supplement,<br>1 May 2021 (2021-05-01), pages S96 CONF<br>20210525 to 20210528 Virtual, Online-27th<br>Annu,<br>ISSN: 1465-3249<br>----- | 13<br>8,9 | |
| A | VICTOR JARAVINE ET AL: "Assessment of<br>cancer and virus antigens for<br>cross-reactivity in human tissues",<br>BIOINFORMATICS,<br>vol. 33, no. 1,<br>10 September 2016 (2016-09-10), pages<br>104-111, XP055675495,<br>GB<br>ISSN: 1367-4803, DOI:<br>10.1093/bioinformatics/btw567<br>* the whole document *<br>-----<br>-/-- | 1-15 | TECHNICAL FIELDS<br>SEARCHED (IPC)<br><br>G16B |

The present search report has been drawn up for all claims

| Place of search | Date of completion of the search | Examiner |
|---|---|---|
| Munich | 12 May 2023 | Mühlbauer, Max |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................................
& : member of the same patent family, corresponding
    document

EPO FORM 1503 03.82 (P04C01)

**page 1 of 3**

**EUROPEAN SEARCH REPORT**

Europäisches Patentamt
European Patent Office
Office européen des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JARAVINE VICTOR ET AL: "Expitope 2.0: a tool to assess immunotherapeutic antigens for their potential cross-reactivity against naturally expressed proteins in human tissues", BMC CANCER, vol. 17, no. 1, 1 December 2017 (2017-12-01), XP93045380, DOI: 10.1186/s12885-017-3854-8 Retrieved from the Internet: URL:http://link.springer.com/content/pdf/10.1186/s12885-017-3854-8.pdf> | 13 | |
| A | * p.3, section "Identification of natural epitopes" p. 3, section "Calculation of the tissue weighted CR-index" * | 1-12,14,15 | |
| A | MOISE LEONARD ET AL: "The two-faced T cell epitope : Examining the host-microbe interface with JanusMatrix", HUMAN VACCINES & IMMUNOTHERAPEUTICS, vol. 9, no. 7, 12 April 2013 (2013-04-12), pages 1577-1586, XP93045458, US ISSN: 2164-5515, DOI: 10.4161/hv.24615 * p. 1578, right column, lines 11-14 * | 1-15 | TECHNICAL FIELDS SEARCHED (IPC) |

-/--

The present search report has been drawn up for all claims

| Place of search | Date of completion of the search | Examiner |
|---|---|---|
| Munich | 12 May 2023 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | Europäisches Patentamt<br>European Patent Office<br>Office européen des brevets | **EUROPEAN SEARCH REPORT** | **Application Number**<br>**EP 22 46 1636** |
|---|---|---|---|

## DOCUMENTS CONSIDERED TO BE RELEVANT

| Category | Citation of document with indication, where appropriate,<br>of relevant passages | Relevant<br>to claim | CLASSIFICATION OF THE<br>APPLICATION (IPC) |
|---|---|---|---|
| T | Pienkowski Victor Murcia ET AL: "ARDitox: platform for the prediction of TCRs potential off-target binding",<br>bioRxiv,<br>12 April 2023 (2023-04-12), pages 1-21,<br>XP093045463,<br>DOI: 10.1101/2023.04.11.536336<br>Retrieved from the Internet:<br>URL:https://www.biorxiv.org/content/biorxiv/early/2023/04/12/2023.04.11.536336.full.pdf<br>[retrieved on 2023-05-09]<br>* the whole document *<br>-----<br> | | |
| A | Nielsen Morten ET AL: "Immunoinformatics: Predicting Peptide-MHC Binding",<br>Annu. Rev. Biomed. Data Sci,<br>25 June 2020 (2020-06-25), pages 57191-215, XP055886158,<br>DOI: 10.1146/annurev-biodatasci-021920-<br>Retrieved from the Internet:<br>URL:https://notablesdelaciencia.conicet.gov.ar/bitstream/handle/11336/140643/CONICET_Digital_Nro.113d08a6-d0a1-4493-a315-da737a446e9f_A.pdf?sequence=2&isAllowed=y<br>[retrieved on 2022-02-01]<br>* the whole document *<br>-----<br> | 1-15 | TECHNICAL FIELDS<br>SEARCHED (IPC) |

The present search report has been drawn up for all claims

| Place of search | Date of completion of the search | Examiner |
|---|---|---|
| Munich | 12 May 2023 | Mühlbauer, Max |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
.....................................................................
& : member of the same patent family, corresponding
    document

EPO FORM 1503 03.82 (P04C01)

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 11017882 B2
- US 2019227063 A1
- WO 2019006427 A1

## Non-patent literature cited in the description

- **KLEIN L ; KYEWSKI B ; ALLEN PM ; HOGQUIST KA.** Positive and negative selection of the T cell repertoire: what thymocytes see (and don't see. *Nat Rev Immunol.,* 16 May 2014, vol. 14 (6), 377-91
- **DHANIK et al.** In-silico discovery of cancer-specific peptide-HLA complexes for targeted therapy. *BMC Bioinformatics,* 2016, vol. 17, 286
- **MOISE, L. ; GUTIERREZ, A. H. ; KIBRIA, F. ; MARTIN, R. ; TASSONE, R. ; LIU, R. ; TERRY, F. ; MARTIN, W. ; DE GROOT, A. S.** iVax: "An integrated toolkit for the selection and optimization of antigense and the design of epitope-driven vaccines. *Human Vaccines & Immunotherapeutics,* 2015, vol. 11 (9), 2312-2321
- **MOISE L ; GUTIERREZ AH ; BAILEY-KELLOGG C ; TERRY F ; LENG Q ; ABDEL HADY KM ; VERBERKMOES NC ; SZTEIN MB ; LOSIKOFF PT ; MARTIN WD.** The two-faced T cell epitope: examining the host-microbe interface with JanusMatrix. *Hum Vaccin Immunother.,* July 2013, vol. 9 (7), 1577-86
- **EKERUCHE-MAKINDE J ; MILES JJ ; VAN DEN BERG HA ; SKOWERA A ; COLE DK ; DOLTON G ; SCHAUENBURG AJ ; TAN MP ; PENTIER JM ; LLEWELLYN-LACEY S.** Peptide length determines the outcome of TCR/peptide-MHCI engagement. *Blood,* 14 February 2013, vol. 121 (7), 1112-23
- *Nucleic Acids Res.,* 08 January 2021, vol. 49 (D1), D480-D489
- **SHEN LX ; BASILION JP ; STANTON VP JR.** Single-nucleotide polymorphisms can cause different structural folds of mRNA. *Proc Natl Acad Sci U S A.,* 06 July 1999, vol. 96 (14), 7871-6
- **KWAK SH ; CHAE J ; CHOI S ; KIM MJ ; CHOI M ; CHAE JH ; CHO EH ; HWANG TJ ; JANG SS ; KIM JI.** Findings of a 1303 Korean whole-exome sequencing study. *Exp Mol Med.,* 14 July 2017, vol. 49 (7), e356
- **SIWEI CHEN ; LAURENT C. FRANCIOLI ; JULIA K. GOODRICH ; RYAN L. COLLINS ; QINGBO WANG ; JESSICA ALFÖLDI ; NICHOLAS A. WATTS ; CHRISTOPHER VITTAL ; LAURA D. GAUTHIER et al.** A genome-wide mutational constraint map quantified from variation in 76,156 human genomes. *bioRxiv 2022.03.20.485034,* https://gnomad.broadinstitute.org
- **ABELIN et al.** Spectrometry Profiling of HLA-Associated Peptidomes in Mono-allelic Cells Enables More Accurate Epitope Prediction. *Immunity.,* 21 February 2017, vol. 46 (2), 315-326
- **DI MARCO et al.** Unveiling the peptide motifs of HLA-C and HLA-G from naturally presented peptides and generation of binding prediction matrices. *J. Immunol.,* 2017, vol. 199, 2639-265110
- **SARKIZOVA et al.** A large peptidome dataset improves HLA class I epitope prediction across most of the human population. *Nat. Biotechnol.,* 2020, vol. 38, 199-209
- **TIMOTHY J O'DONNELL ; ALEX RUBINSTEYN ; MARIA BONSACK ; ANGELIKA 8 RIEMER ; URI LASERSON ; JEFF HAMMERBACHER.** *MHCflurry: Open-Source Class I MHC Binding Affinity Prediction*
- **BORDER EC ; SANDERSON JP ; WEISSENSTEINER T ; GERRY AB ; PUMPHREY NJ.** Affinity-enhanced T-cell receptors for adoptive T-cell therapy targeting MAGE-A 10: strategy for selection of an optimal candidate. *Oncoimmunology*
- **BLABER M ; ZHANG XJ ; MATTHEWS BW.** Structural basis of amino acid alpha helix propensity. *Science,* 11 June 1993, vol. 260 (5114), 1637-40
- **BIOU V ; GIBRAT JF ; LEVIN JM ; ROBSON B ; GARNIER J.** Secondary structure prediction: combination of three different methods. *Protein Eng.,* September 1988, vol. 2 (3), 185-91
- **TSAI J ; TAYLOR R ; CHOTHIA C ; GERSTEIN M.** The packing density in proteins: standard radii and volumes. *J Mol Biol.,* 02 July 1999, vol. 290 (1), 253-66

- **CEDANO J ; ALOY P ; PÉREZ-PONS JA ; QUEROL E.** Relation between amino acid composition and cellular location of proteins. *J Mol Biol.,* 28 February 1997, vol. 266 (3), 594-600
- **NAKASHIMA H ; NISHIKAWA K.** The amino acid composition is different between the cytoplasmic and extracellular sides in membrane proteins. *FEBS Lett.,* 01 June 1992, vol. 303 (2-3), 141-6
- **MIYAZAWA S ; JERNIGAN RL.** Self-consistent estimation of inter-residue protein contact energies based on an equilibrium mixture approximation of residues. *Proteins,* 01 January 1999, vol. 34 (1), 49-68
- **SAHA I ; MAULIK U ; BANDYOPADHYAY S ; PLEWCZYNSKI D.** Fuzzy clustering of physico-chemical and biochemical properties of amino acids. *Amino Acids.,* August 2012, vol. 43 (2), 583-94
- GTEx Consortium. The Genotype-Tissue Expression (GTEx) project. *Nat Genet.,* June 2013, vol. 45 (6), 580-5
- **UHLÉN M et al.** Tissue-based map of the human proteome. *Science,* 23 January 2015, vol. 347 (6220), 1260419
- **PEDREGOSA et al.** Scikit-learn: Machine Learning in Python. *JMLR,* 2011, vol. 12, 2825-2830
- **REBACK, J. ; MCKINNEY, W. et al.** *Pandas-Dev/Pandas: Pandas 1.2.2, Zenodo [code,* 2021, https://doi.org/10.5281/zenodo.4524629
- **HARRIS, C.R. ; MILLMAN, K.J. ; VAN DER WALT, S.J. et al.** Array programming with NumPy. *Nature,* 2020, vol. 585, 357-362
- **WICKHAM H ; FRANÇOIS R ; HENRY L ; MÜLLER K.** *dplyr: A Grammar of Data Manipulation,* 2022
- **WICKHAM H.** ggplot2: Elegant Graphics for Data Analysis. Springer-Verlag, 2016
- **PAGÈS H.** *BSgenome: Software infrastructure for efficient representation of full genomes and their SNPs,* 2022
- **VITA R ; MAHAJAN S ; OVERTON JA ; DHANDA SK ; MARTINI S ; CANTRELL JR ; WHEELER DK ; SETTE A ; PETERS B.** The Immune Epitope Database (IEDB): 2018 update. *Nucleic Acids Res.,* 24 October 2018
- **KOSTER J ; PLASTERK RHA.** A library of Neo Open Reading Frame peptides (NOPs) as a sustainable resource of common neoantigens in up to 50% of cancer patients. *Sci Rep.,* 29 April 2019, vol. 9 (1), 6577
- **MORGAN RA ; CHINNASAMY N ; ABATE-DAGA D ; GROS A ; ROBBINS PF ; ZHENG Z ; DUDLEY ME ; FELDMAN SA ; YANG JC ; SHERRY RM.** Cancer regression and neurological toxicity following anti-MAGE-A3 TCR gene therapy. *J Immunother.,* February 2013, vol. 36 (2), 133-51
- **LINETTE GP ; STADTMAUER EA ; MAUS MV ; RAPOPORT AP ; LEVINE BL ; EMERY L ; LITZKY L ; BAGG A ; CARRENO BM ; CIMINO PJ.** Cardiovascular toxicity and titin cross-reactivity of affinity-enhanced T cells in myeloma and melanoma. *Blood,* 08 August 2013, vol. 122 (6), 863-71
- **CAI L ; CARABALLO GALVA LD ; PENG Y ; LUO X ; ZHU W ; YAO Y ; JI Y ; HE Y.** Preclinical Studies of the Off-Target Reactivity of AFP 158 -Specific TCR Engineered T Cells. *Front Immunol.,* 27 April 2020, vol. 11, 607
- **STADTMAUER EA ; FAITG TH ; LOWTHER DE ; BADROS AZ ; CHAGIN K ; DENGEL K ; LYENGAR M ; MELCHIORI L ; NAVENOT JM ; NORRY E.** Long-term safety and activity of NY-ESO-1 SPEAR T cells after autologous stem cell transplant for myeloma. *Blood Adv.,* 09 July 2019, vol. 3 (13), 2022-2034